# EUROPEAN PATENT SPECIFICATION

(11) **EP 4 330 935 B1**
(45) Date of publication and mention of the grant of the patent: **06.08.2025**
(21) Application number: 22914970.3
(22) Date of filing: 28.12.2022
(51) Int. Cl.: G06V 40/10, G06N 20/00, G06V 10/82

(54) **SYSTEMS AND METHODS FOR MEDICAL IMAGING**
SYSTEME UND VERFAHREN ZUR MEDIZINISCHEN BILDGEBUNG
SYSTÈMES ET PROCÉDÉS D'IMAGERIE MÉDICALE

(30) Priority: 29.12.2021 CN 202111649411; 31.12.2021 CN 202111679878
(43) Date of publication of application: 06.03.2024
(73) Proprietor: Shanghai United Imaging Healthcare Co., Ltd., Shanghai 201807 (CN)
(72) Inventor: XU, Liang, Shanghai 201807 (CN); ZHONG, Jian, Shanghai 201807 (CN); FENG, Juan, Shanghai 201807 (CN); MA, Yan'ge, Shanghai 201807 (CN); YUAN, Zhou, Shanghai 201807 (CN)
(74) Representative: Wang, Bo
(86) International application number: PCT/CN2022/143012
(87) International publication number: WO 2023/125720

(56) References cited:
- CN-A- 110 536 641
- CN-A- 111 339 846
- CN-A- 111 462 115
- CN-A- 111 462 115
- CN-A- 112 203 721
- CN-A- 113 177 928
- CN-A- 114 299 547
- US-A1- 2013 274 537

## Description

### CROSS-REFERENCE TO RELATED APPLICATIONS

### TECHNICAL FIELD

The present disclosure relates to medical technology, and in particular, to systems and methods for medical imaging.

### BACKGROUND

Medical imaging technology has been widely used for creating images of interior of a patient's body for, e.g., clinical examinations, medical diagnosis, and/or treatment purposes. CN111462115A (SHANGHAI UNITED IMAGING HEALTHCARE CO LTD) discloses a medical image display method and device, computer equipment and a readable storage medium, including the automatic determination of a region of interest in the scanned image.

### SUMMARY

The invention is defined in the set of claims.

### BRIEF DESCRIPTION OF THE DRAWINGS

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings, and wherein:
FIG. 1 is a schematic diagram illustrating an exemplary medical system 100 according to some embodiments of the present disclosure;
FIG. 2 is a block diagram illustrating exemplary processing device according to some embodiments of the present disclosure;
FIG. 3 is a flowchart illustrating an exemplary process for positioning a medical device according to some embodiments of the present disclosure;
FIG. 4 is a schematic diagram for determining an image region according to some embodiments of the present disclosure;
FIG. 5 is a schematic diagram illustrating an exemplary body part boundary recognition model according to some embodiments of the present disclosure;
FIG. 6 is a schematic diagram illustrating an exemplary feature point recognition model according to some embodiments of the present disclosure;
FIG. 7 is a flowchart illustrating an exemplary process for determining a radiation region of a radioactive medical device according to some embodiments of the present disclosure;
FIG. 8 is a schematic diagram illustrating an exemplary second optical image according to some embodiments of the present disclosure; and
FIG. 9 is a flowchart illustrating an exemplary process 900 for generating a position information determination model or a radiation region information determination model according to some embodiments of the present disclosure.

### DETAILED DESCRIPTION

In the following detailed description, numerous specific details are set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high level, without detail, in order to avoid unnecessarily obscuring aspects of the present disclosure.

In the following detailed description, numerous specific details are set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high level, without detail, in order to avoid unnecessarily obscuring aspects of the present disclosure.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this specification, specify the presence of stated features, integers, steps, operations, elements, and/or components, but do not preclude the presence or addition of one or more other features, integers, steps, operations, elements, components, and/or groups thereof.

It will be understood that the term "system," "engine," "unit," "module," and/or "block" used herein are one method to distinguish different components, elements, parts, sections or assembly of different levels in ascending order. However, the terms may be displaced by another expression if they achieve the same purpose.

Generally, the word "module," "unit," or "block," as used herein, refers to logic embodied in hardware or firmware, or to a collection of software instructions. In general, the modules/units/blocks described herein refer to logical modules/units/blocks that may be combined with other modules/units/blocks or divided into sub-modules/sub-units/sub-blocks despite their physical organization or storage. The description may be applicable to a system, an engine, or a portion thereof.

It will be understood that when a unit, engine, module, or block is referred to as being "on," "connected to," or "coupled to," another unit, engine, module, or block, it may be directly on, connected or coupled to, or communicate with the other unit, engine, module, or block, or an intervening unit, engine, module, or block may be present, unless the context clearly indicates otherwise. As used herein, the term "and/or" includes any and all combinations of one or more of the associated listed items. The term "pixel" and "voxel" in the present disclosure are used interchangeably to refer to an element of an image. An anatomical structure shown in an image of a subject (e.g., a patient) may correspond to an actual anatomical structure existing in or on the subject's body. For example, a body part shown in an image may correspond to an actual body part existing in or on the subject's body, and a feature point in an image may correspond to an actual feature point existing in or on the subject's body. For the convenience of descriptions, an anatomical structure shown in an image and its corresponding actual anatomical structure are used interchangeably. For example, the chest of the subject refers to the actual chest of the subject or a region representing the chest in an image of the subject.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present disclosure. It is understood that the drawings are not to scale.

Before a medical scan or treatment of a subject starts, a medical device (e.g., an X-ray imaging device) needs to be positioned, so that a target region of the subject can be imaged and/or treated effectively. For example, if a radioactive medical device is used to perform the medical scan of the subject, the radioactive medical device needs to be positioned before the medical scan so that radioactive rays accurately cover the target region of the subject during the medical scan. Conventional medical scan positioning approaches often involve a lot of human intervention, which have limited efficiency and accuracy. For example, an initial target position of the medical device is determined based on an examination protocol and other relevant information (e.g., an average height of the human body), and then a user (e.g., a doctor, an operator, a technician, etc.) adjusts the initial target position to an appropriate position based on visual inspection. Thus, it may be desirable to develop systems and methods for positioning a medical device with an improved efficiency and accuracy.

An aspect of the present disclosure relates to systems and methods for medical imaging. The systems may obtain a first optical image of the target subject that includes a target region to be scanned or treated by a medical device. The systems also may identify one or more body part boundaries and one or more feature points of the target subject from the first optical image using at least one target recognition model. The systems may identify an image region corresponding to the target region of the target subject from the first optical image based on the one or more body part boundaries and one or more feature points. At least one first edge of the image region may be determined based on the one or more body part boundaries, and at least one second edge of the image region may be determined based on the one or more feature points.

In some embodiments, the systems may further determine whether a positioning procedure of the medical device can be started based on the image region. In response to determining that the positioning procedure of the medical device can be started, the systems may determine a positioning target of the medical device according to the image region, and perform the positioning procedure of the medical device according to the positioning target of the medical device. Compared with the conventional medical scan positioning approaches, the methods and systems of the present disclosure may be implemented with reduced or minimal or without user intervention, which is more efficient and accurate by, e.g., reducing the workload of a user, cross-user variations, and the time needed for the positioning.

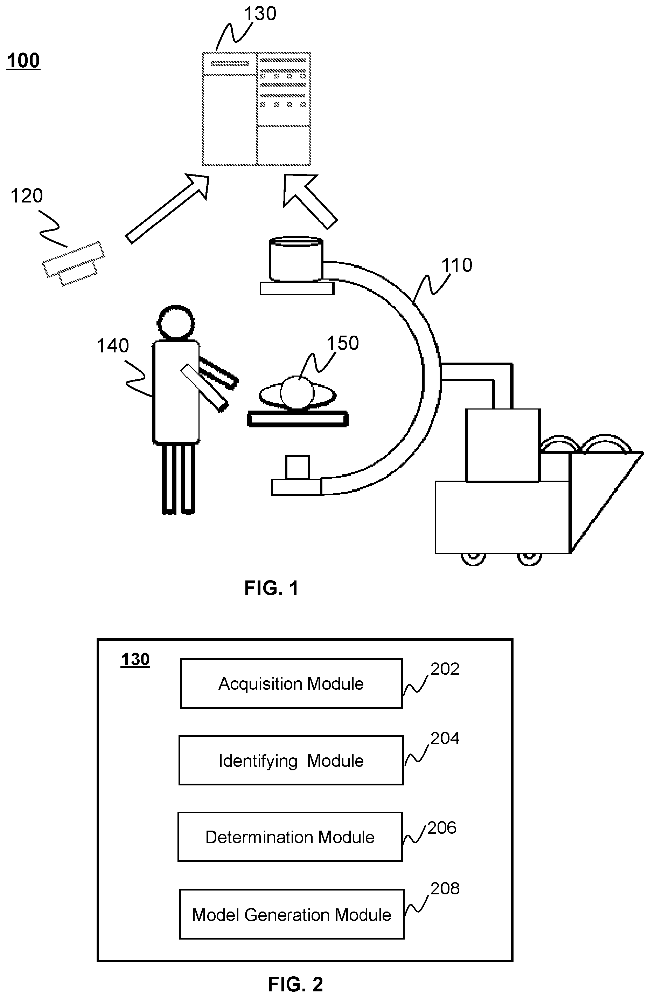

FIG. 1 is a schematic diagram illustrating an exemplary medical system 100 according to some embodiments of the present disclosure. As shown in FIG. 1, the medical system 100 may include a medical device 110, an image acquisition device 120, and a processing device 130. In some embodiments, the components of the medical system 100 may be connected to and/or communicate with each other via a wireless connection, a wired connection, or a combination thereof. The connections between the components in the medical system 100 may be variable. For example, the medical device 110 may be connected to the processing device 130 through a network. As another example, the medical device 110 may be connected to the processing device 130 directly.

The medical device 110 may be configured to scan and/or treat a subject (e.g., a subject 150 shown in FIG. 1). Merely by way of example, the medical device 110 may generate or provide medial image data relating to the subject via scanning the subject. The medial image data relating to the subject may be used for generating an anatomical image of the subject. The anatomical image may illustrate an internal structure of the subject. In some embodiments, the subject may include a biological subject and/or a non-biological subject. For example, the subject may include a specific portion of a body, such as the head, the thorax, the abdomen, or the like, or a combination thereof. As another example, the subject may be a man-made composition of organic and/or inorganic matters that are with or without life. In some embodiments, the medical system 100 may include modules and/or components for performing imaging and/or related analysis.

In some embodiments, the medical device 110 may be a non-invasive biomedical medical imaging device for disease diagnostic or research purposes. The medical device 110 may include a single modality scanner and/or a multi-modality scanner. The single modality scanner may include, for example, an ultrasound scanner, an X-ray scanner, a computed tomography, CT, scanner, a magnetic resonance imaging, MRI, scanner, an ultrasonography scanner, a positron emission tomography, PET, scanner, an optical coherence tomography, OCT, scanner, an ultrasound, US, scanner, an intravascular ultrasound, IVUS, scanner, a near infrared spectroscopy, NIRS, scanner, a far infrared, FIR, scanner, or the like, or any combination thereof. The multi-modality scanner may include, for example, an X-ray imaging-magnetic resonance imaging, X-ray-MRI, scanner, a positron emission tomography-X-ray imaging, PET-X-ray, scanner, a single photon emission computed tomography-magnetic resonance imaging, SPECT-MRI, scanner, a positron emission tomography- computed tomography, PET-CT, scanner, a digital subtraction angiography-magnetic resonance imaging, DSA-MRI, scanner, etc. It should be noted that the scanner described above is merely provided for illustration purposes, and not intended to limit the scope of the present disclosure. The term "imaging modality" or "modality" as used herein broadly refers to an imaging method or technology that gathers, generates, processes, and/or analyzes imaging information of a subject.

In some embodiments, the medical device 110 may include a radiotherapy, RT, device, such as a conformal radiation therapy device, an image-guided radiation therapy, IGRT, device, an intensity-modulated radiation therapy, IMRT, device, an intensity-modulated arc therapy, IMAT, device, an emission guided radiation therapy, EGRT, or the like. The RT device may be configured to deliver a radiotherapy treatment to the subject. For example, the RT device may deliver one or more radiation beams to a treatment region (e.g., a tumor) of the subject for causing an alleviation of the subject's symptom. A radiation beam may include a plurality of radiation beamlets.

The image acquisition device 120 may be configured to capture an optical image of the subject, which may illustrate an external body surface of the subject. For example, the image acquisition device 120 may be configured to capture one or more optical images of the subject during the medical scan or the treatment of the subject performed by the medical device 110. The image acquisition device 120 may be and/or include any suitable device capable of capturing optical images of subjects located in a field of view of the image acquisition device 120. For example, the image acquisition device 120 may include a camera (e.g., a digital camera, an analog camera, a binocular camera, etc.), a red-green-blue, RGB, sensor, an RGB-depth, RGB-D, sensor, a time-of-flight, TOF, camera, a depth camera, a structure light camera, a laser radar, or the like, or any combination thereof. In some embodiments, the optical image(s) captured by the image acquisition device 120 may include a still image, a dynamic image, a real-time monitoring image, or the like. The optical image(s) may be images of the subject from multiple angles, a panoramic image, or a partial image. The optical image(s) may include a two-dimensional image, a three-dimensional image, a four-dimensional image (e.g., including a series of images over time), or the like. The optical image(s) may be a black and white image, a color image, an optical image, or a combination of both.

In some embodiments, the image capturing device 160 may be a device independent from the medical device 110 as shown in FIG. 1. For example, the image capturing device 160 may be a camera mounted on the ceiling in an examination room where the medical device 110 is located or out of the examination room. Alternatively, the image capturing device 160 may be integrated into or mounted on the medical device 110 (e.g., the gantry 111).

The processing device 130 may be configured to process data and/or information obtained from one or more components (e.g., the medical device 110, the image acquisition device 120, etc.) of the medical system 100. In some embodiments, before a scan or a treatment is performed on a target region of the subject, the processing device 130 may determine position information of the target region for guiding the positioning of the medical device 110 so that the target region can be scanned or treated accurately. For example, the processing device 130 may obtain a first optical image of a target subject (e.g., the subject 150) captured by the image acquisition device 120. The target subject may include a target region to be scanned or treated by the medical device 110. The processing device 130 may identify at least one body part boundary and at least one feature point of the target subject from the first optical image using at least one target recognition model. The processing device 130 may further identify an image region corresponding to the target region of the subject from the first optical image based on the at least one body part boundary and the at least one feature point. At least one first edge of the image region may be determined based on the at least one body part boundary, and at least one second edge of the image region may be determined based on the at least one feature point.

In some embodiments, if the medical device 110 is radioactive medical device, the medical workers may need to be protected from radiation damage. For example, during the scan or the treatment performed by the medical device 110 on the target subject, the processing device 130 may obtain a second optical image indicating the scene of the scan or the treatment. The processing device 130 may determine first information of one or more medical workers 140 who participate in the scan or the treatment and second position information of a radiation region of the radioactive medical device based on the second optical image. The processing device 130 may further determine whether at least one of the one or more medical workers 140 need to change positions based on the first information and the second position information.

In some embodiments, the processing device 130 may be a single server or a server group. The server group may be centralized or distributed. In some embodiments, the processing device 130 may be local or remote. Merely for illustration, only one processing device 130 is described in the medical system 100. However, it should be noted that the medical system 100 in the present disclosure may also include multiple processing devices. Thus operations and/or method steps that are performed by one processing device 130 as described in the present disclosure may also be jointly or separately performed by the multiple processing devices. For example, if in the present disclosure the processing device 130 of the medical system 100 executes both process A and process B, it should be understood that the process A and the process B may also be performed by two or more different processing devices jointly or separately in the medical system 100 (e.g., a first processing device executes process A and a second processing device executes process B, or the first and second processing devices jointly execute processes A and B).

This description is intended to be illustrative, and not to limit the scope of the present disclosure. Many alternatives, modifications, and variations will be apparent to those skilled in the art. The features, structures, methods, and characteristics of the exemplary embodiments described herein may be combined in various ways to obtain additional and/or alternative exemplary embodiments. However, those variations and modifications do not depart the scope of the present disclosure. Merely by way of example, the medical system 100 may include one or more additional components and/or one or more components described above may be omitted. For example, the medical system 100 may include a storage device configured to store data, instructions, and/or any other information. For example, the storage device may store data obtained from the medical device 110, the image acquisition device 120, and the processing device 130. In some embodiments, the storage device may store data and/or instructions that the processing device 130 may execute or use to perform exemplary methods described in the present disclosure. As another example, the medical system 100 may include a network. The network may include any suitable network that can facilitate the exchange of information and/or data for the medical system 100. In some embodiments, one or more components of the medical system 100 (e.g., the medical device 110, the image acquisition device 120, the processing device 130, the storage device, etc.) may communicate information and/or data with one or more other components of the medical system 100 via the network.

FIG. 2 is a block diagram illustrating exemplary processing device 130 according to some embodiments of the present disclosure.

As shown in FIG. 2, the processing device 130 may include an acquisition module 202, an identifying module 204, a determination module 206, and a model generation module 208. As described in FIG. 1, the medical system 100 in the present disclosure may also include multiple processing devices, and the acquisition module 202, the identifying module 204, the determination module 206, and the model generation module 208 may be components of different processing devices.

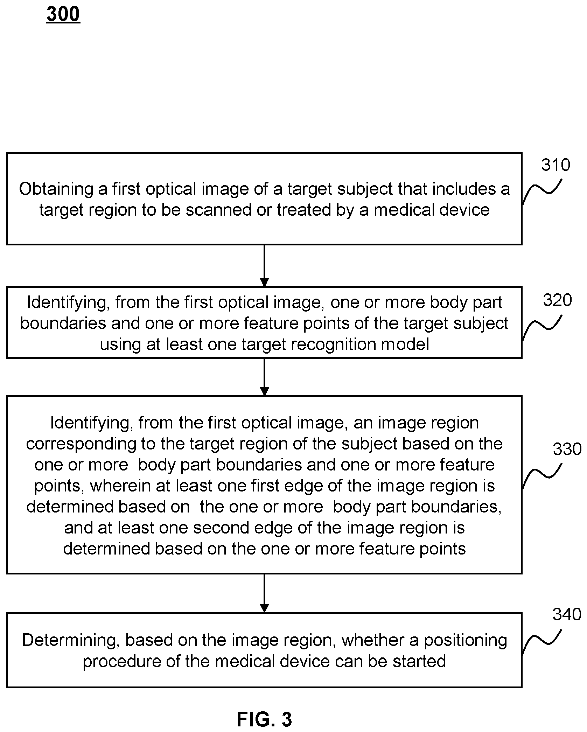

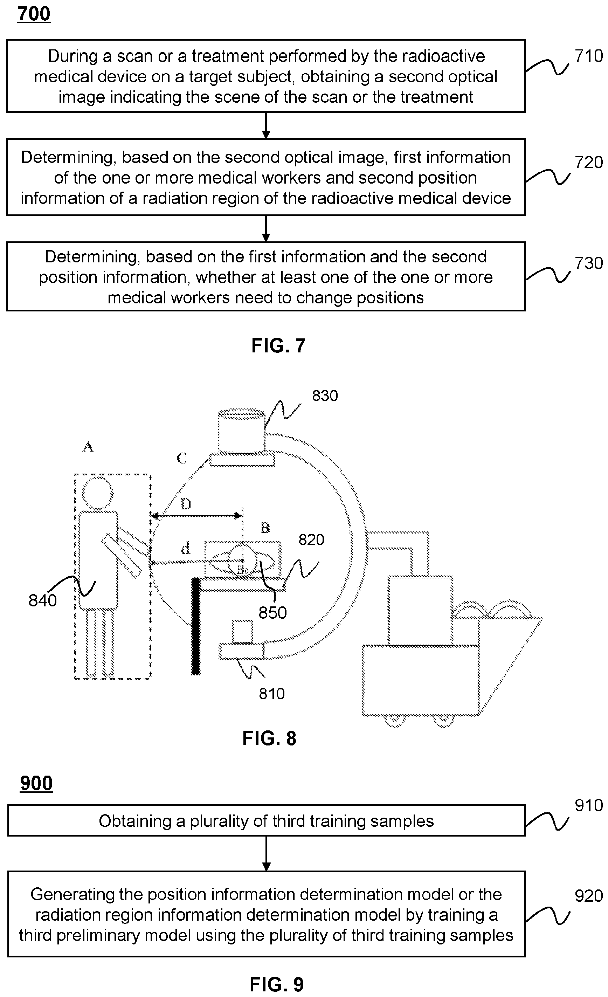

The acquisition module 202 may be configured to obtain information relating to the medical system 100. For example, the acquisition module 202 may obtain a first optical image of a target subject that includes a target region to be scanned or treated by a medical device. More descriptions regarding the obtaining of the first optical image may be found elsewhere in the present disclosure. See, e.g., operation 310 in FIG. 3, and relevant descriptions thereof. As another example, the acquisition module 220 may be configured to obtain a second optical image indicating the scene of the scan or the treatment. More descriptions regarding the obtaining of the second optical image may be found elsewhere in the present disclosure. See, e.g., operation 710 in FIG. 7, and relevant descriptions thereof.

The identifying module 204 may be configured to identify one or more body part boundaries and one or more feature points of the target subject from the first optical image using at least one target recognition model. More descriptions regarding the identifying of the one or more body part boundaries and the one or more feature points of the target subject may be found elsewhere in the present disclosure. See, e.g., operation 320 in FIG. 3, and relevant descriptions thereof.

The identifying module 204 may be also configured to identify an image region corresponding to the target region of the target subject from the first optical image based on the one or more body part boundaries and one or more feature points. In some embodiments, at least one first edge of the image region may be determined based on the one or more body part boundaries, and at least one second edge of the image region may be determined based on the one or more feature points. More descriptions regarding the identifying of the image region corresponding to the target region may be found elsewhere in the present disclosure. See, e.g., operation 330 in FIG. 3, and relevant descriptions thereof.

The determination module 206 may be configured to determine whether a positioning procedure of the medical device can be started based on the image region. More descriptions regarding the determining of whether a positioning procedure of the medical device can be started may be found elsewhere in the present disclosure. See, e.g., operation 340 in FIG. 3, and relevant descriptions thereof.

The determination module 206 may be configured to determine first information of one or more medical workers who participate in the scan or the treatment and second position information of a radiation region of the radioactive medical device based on the optical image. More descriptions regarding the determining of the first information and the second position information may be found elsewhere in the present disclosure. See, e.g., operation 720 in FIG. 7, and relevant descriptions thereof.

The determination module 206 may be configured to determine whether at least one of the one or more medical workers need to change positions based on the first information and the second position information. More descriptions regarding the determining of whether at least one of the one or more medical workers need to change positions may be found elsewhere in the present disclosure. See, e.g., operation 730 in FIG. 7, and relevant descriptions thereof.

The model generation module 208 may be configured to obtain data used to train one or more models, such as a body part boundary recognition model, a feature point recognition model, a position information determination model, a radiation region information determination model, disclosed in the present disclosure. For example, the model generation module 208 may be configured to obtain a plurality of training samples and a preliminary model, and generate the one or more models by model training. More descriptions regarding the generation of the one or more models may be found elsewhere in the present disclosure may be found elsewhere in the present disclosure. See, e.g., operation 320 in FIG. 3, FIG. 9, and relevant descriptions thereof.

It should be noted that the above description is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, any one of the modules may be divided into two or more units. For instance, the acquisition module 202 may be divided into two units configured to acquire different data. In some embodiments, the processing device 130 may include one or more additional modules, such as a storage module (not shown) for storing data.

FIG. 3 is a flowchart illustrating an exemplary process 300 for positioning a medical device according to some embodiments of the present disclosure.

In 310, the processing device 130 (e.g., the acquisition module 202) may obtain a first optical image of a target subject that includes a target region to be scanned or treated by a medical device.

The target subject may include any subject that needs to be scanned or treated. The medical device may be any suitable medical device (e.g., the medical device 110) configured to scan and/or treat the target subject. As used herein, the target region of the target subject refers to a region of the target subject that needs to receive the scan and/or treatment for clinical examinations, medical diagnosis, and/or treatment purposes. For example, the target region may include the lungs of a patient including nodules that need to be removed. As another example, the target region may include a region of a patient including a tumor that need to receive radiotherapy. For illustration purposes, the positioning of the medical device before a scan of the target subject is described hereinafter.

In some embodiments, before the scan, one or more optical images of the target subject may be captured by an image acquisition device (e.g., the image acquisition device 120). In some embodiments, the optical image(s) may include a plurality of optical images. The target subject may remain stationary when the optical images are captured, and the position of the target subject may remain unchanged. In some embodiments, the target subject may move (e.g., walk, rotate, etc.) or shake (e.g., shake left and right, shake up and down, etc.) when the optical images are captured, and the position of the target subject may change with the movement or shaking.

In some embodiments, the position of the target subject may refer to a position of the target subject in an optical image. For example, the position of the target subject may be represented by coordinates of the target subject or a certain point on the target subject in a coordinate system (e.g., whose origin is located at a starting point pixel in the lower left corner of the optical image). In some embodiments, the position of the target subject may refer to a position of the target subject in space, for example, a position of the target subject relative to a reference object (e.g., a point or an object in space). For example, the position of the target subject may be represented by coordinates of the target subject or a certain point on the target subject in a coordinate system (e.g., whose origin is located at the reference object or a point on the reference object).

In some embodiments, the initial acquired optical image(s) may be subjected to a preprocess operation to facilitate subsequent processing of the optical image(s). Exemplary preprocess operations may include an image normalization (e.g., a resolution normalization, a size normalization, etc.), an image filling (e.g., an image pixel filling, etc.), an image flipping, an image rotation, etc.

In some embodiments, the processing device 130 may select one optical image from the optical image(s) as the first optical image. For example, the processing device 130 may select the latest acquired optical image from the optical image(s) as the first optical image.

In 320, the processing device 130 (e.g., the identifying module 204) may identify, from the first optical image, one or more body part boundaries and one or more feature points of the target subject using at least one target recognition model.

In some embodiments, the target subject may include one or more body parts. For example, a person may include multiple body parts such as the head, the neck, the torso, the chest, the waist, buttocks, the left arm, the right arm, hands, the left lower limb, the right lower limb, and feet. As another example, a dog may include multiple body parts such as the head, the neck, the chest, the left forelimb, the right forelimb, the left hindlimb, the right hindlimb, and the tail.

A body part boundary refers to a boundary of a body part. In some embodiments, a region in the first optical image that encloses a body part of the target subject (also referred to as a region corresponding to the body part) may be identified using a bounding box, that is, the boundary of the body part of the target subject may be identified using the bounding box. In other words, the body part boundary may be represented as a bounding box. The bounding box may have the shape of a square, a rectangle, a triangle, a polygon, a circle, an ellipse, an irregular shape, or the like. In some embodiments, each of the one or more body parts may correspond to one bounding box. In some embodiments, the one or more body parts of the target subject and the corresponding regions in the first optical image may be set according to actual experience or needs.

In some embodiments, the at least one target recognition model may include a body part boundary recognition model. The processing device 130 may identify the one or more body part boundaries of the target subject from the first optical image using the body part boundary recognition model. Merely by way of example, the first optical image may be input into the body part boundary recognition model, and the body part boundary recognition model may output one or more bounding boxes of the body part(s) of the target subject and/or information relating to the one or more bounding boxes. In some embodiments, different body part boundaries of the target subject may be identified using different body part boundary recognition models. For example, body part boundaries of the chest and the head of the target subject may be identified using a first body part boundary recognition model and a second body part boundary recognition model, respectively.

In some embodiments, the information relating to a bounding box may include classification information of the bounding box. The classification information of the bounding box may indicate a classification of the body part enclosed by the bounding box. For example, the classification information of a bounding box may indicate that the body part enclosed by the bounding box is the chest.

In some embodiments, the information relating to a bounding box may include position information of the bounding box. Exemplary position information of the bounding box may include coordinates of a center point of the bounding box, a length and a width of the bounding box, or the like, or any combination thereof.

In some embodiments, the information relating to a bounding box may include confidence coefficient information corresponding to the bounding box. The confidence coefficient information corresponding to the bounding box may include a confidence coefficient indicating a reliability that the region of the first optical image enclosed by the bounding box is a foreground region or a background region. As used herein, a foreground region in the first optical image includes one or more regions of interest in the first optical image, such as regions corresponding to one or more body parts of the target subject. A background region refers to regions other than the regions of interest in the first optical image, such as regions corresponding to an instrument, a wall. In some embodiments, the confidence coefficient may be represented in various manners, such as a score, a probability, or the like.

In some embodiments, the body part boundary recognition model may include a deep learning model, such as a Neural Network, NN, model, a Deep Neural Network, DNN, model, a Convolutional Neural Network, CNN, model, a Recurrent Neural Network, RNN, model, a Feature Pyramid Network, FPN, model, etc. Exemplary CNN models may include a V-Net model, a U-Net model, a Link-Net model, or the like, or any combination thereof. In some embodiments, the body part boundary recognition model may include a YOLO model, such as YOLOv1 model, YOLOv2 model, YOLOv3 model, YOLOv4 model, YOLOv5 model, or the like.

In some embodiments, the body part boundary recognition model may include a backbone network. The backbone network may be configured to generate feature maps by extracting image features from the first optical image. The backbone network may include various networks for extracting image features from an image. Exemplary backbone networks may include a CNN, a visual geometry group, VGG, network, a backbone network in YOLO model, a feature extraction network in Faster Region-CNN, or the like.

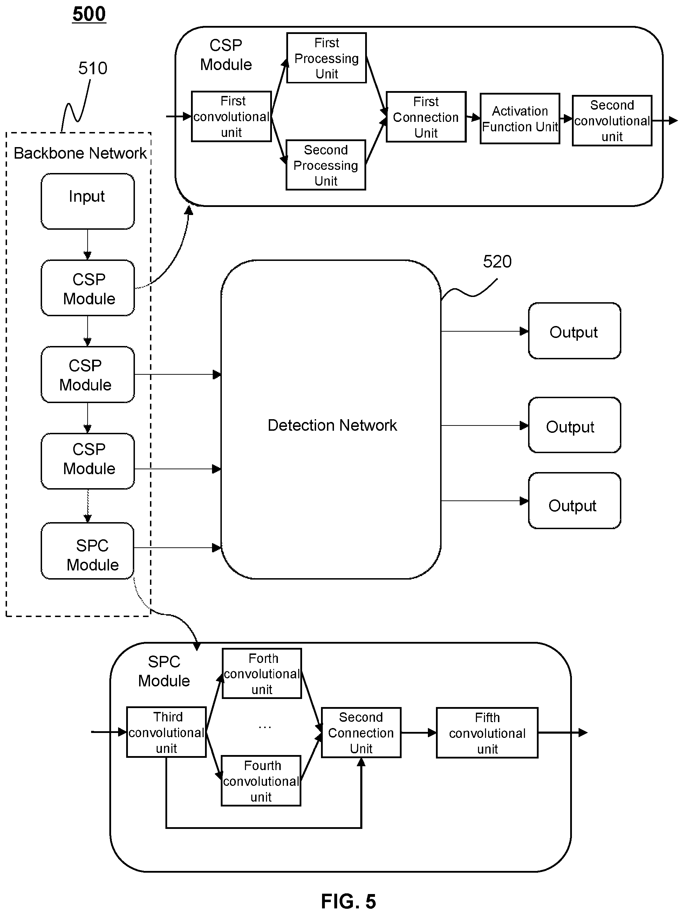

In some embodiments, the body part boundary recognition model may include a detection network. The detection network may be configured to determine information associated with the one or more body part boundaries based on the feature maps extracted by the backbone network. For example, the feature maps extracted by the backbone network may be input into the detection network, and the detection network may output the one or more bounding boxes of the body part(s) of the target subject and/or the information relating to the one or more bounding boxes. The detection network may include various networks for obtaining one or more bounding boxes. Exemplary detection networks may include a CNN, a head network in a YOLO model, a head network and a neck network in a YOLO model, a target detection network in a Faster Region-CNN, or the like. More descriptions for the body part boundary recognition model may be found elsewhere in the present disclosure (e.g., FIG. 5 and the descriptions thereof).

In some embodiments, the processing device 130 may obtain the body part boundary recognition model from one or more components of the medical system 100 or an external source via a network. For example, the body part boundary recognition model may be previously trained by a computing device (e.g., the processing device 130), and stored in a storage device of the medical system 100. The processing device 130 may access the storage device and retrieve the body part boundary recognition model. In some embodiments, the body part boundary recognition model may be generated according to a machine learning algorithm.

Merely by way of example, the body part boundary recognition model may be trained according to a supervised learning algorithm by the processing device 130 or another computing device (e.g., a computing device of a vendor of the body part boundary recognition model). The processing device 130 may obtain a plurality of first training samples and a first preliminary model. Each first training sample may include a first sample optical image of a first sample subject and a first training label. The first training label may include one or more sample bounding boxes enclosing one or more sample body parts of the first sample subject in the first sample optical image and/or sample information relating to the one or more sample bounding boxes. In some embodiments, the sample information relating to a sample bounding box may include sample position information of the sample bounding box, sample confidence coefficient information corresponding to the sample bounding box, and sample classification information relating to each sample body part boundary. In some embodiments, the sample position information, the sample confidence coefficient information, and the sample classification information may be similar to the aforementioned position information, confidence coefficient information, and classification information of a bounding box, respectively.

In some embodiments, the body part boundary recognition model may be generated by training the first preliminary model using the plurality of first training samples according to a first total loss function. The first total loss function may be used to measure a discrepancy between a result predicted by the first preliminary model and the corresponding first training table. In some embodiments, the first total loss function may include one or more of a first loss function, a second loss function, or a third loss function. The first loss function may be configured to evaluate a predicted result associated with the sample position information. Specifically, the first loss function may be used to measure a discrepancy between position information predicted by the first preliminary model and the sample position information. The second loss function may be configured to evaluate a predicted result associated with the sample confidence coefficient information. Specifically, the second loss function may be used to measure a discrepancy between first confidence coefficient information predicted by the first preliminary model and sample confidence coefficient information. The third loss function may be configured to evaluate a predicted result associated with the sample classification information. Specifically, the third loss function may be used to measure a discrepancy between the classification information predicted by the first preliminary model and the first sample classification information. In this way, the body part boundary recognition model may have a relatively high accuracy.

The optimization objective of the training of the first preliminary model may be that the first total loss function satisfies a first preset condition (e.g., being smaller than a threshold value) by adjusting parameters of the first preliminary model. For example, the optimization objective of the training of the first preliminary model may be that the value of the first total loss function is smaller than a certain value.

In some embodiments, the one or more feature points may be identified from the first optical image. As used herein, a feature point refers to a point in the first optical image that represents a point of interest or a critical point of the target subject. For example, the feature points may include one or more feature points on the head (e.g., feature points on the back of the head, etc.), one or more feature points on the neck (e.g., feature points on the middle of the neck, etc.), one or more feature points on the waist (e.g., feature points on the lumbar vertebra, etc.), or the like. In some embodiments, the feature point(s) may be set according to actual experience or needs.

In some embodiments, the at least one target recognition model may include a feature point recognition model. The processing device 130 may identify the one or more feature points of the target subject from the first optical image using the feature point recognition model. Merely by way of example, the first optical image may be input into the feature point recognition model, and the feature point recognition model may output the one or more feature points of the target subject and/or information relating to the one or more feature points. In some embodiments, the information relating to a feature point may include classification information of the feature point. In some embodiments, the classification information of the feature point may indicate a body part that the feature point belongs to. For example, a feature point that is located on the waist may be classified as a waist feature point. In some embodiments, feature points that are located on different body parts may be identified using different feature point recognition models. For example, chest feature points and head feature points may be identified using a chest feature point recognition model and a head feature point recognition model, respectively.

In some embodiments, the feature point recognition model may output a heatmap indicating a probability that each point in the first optical image is a feature point of the target subject. The processing device 130 may determine the one or more feature points of the target subject based on the heatmap. For example, the processing device 130 may designate one or more points whose probabilities are greater than a probability threshold (e.g., 90%) as the feature point(s) of the target subject.

In some embodiments, the processing device 130 may determine a second optical image including the body part(s) of the target subject based on the one or more body part boundaries and the first optical image. For example, the target subject may be a specific person, and bounding boxes of body parts (e.g., the head, the neck, the torso, the chest, the waist, buttocks, the left arm, the right arm, hands, the left lower limb, the right lower limb, and feet, etc.) of the specific person may be determined, that is the body part boundaries of the specific person may be determined. An approximate contour of the specific person may be determined according to the bounding boxes (body part boundaries) of the specific person. Then, a portion of the first optical image corresponding to the specific person may be segmented from the first optical image according to the approximate contour of the specific person, and be designated as the second optical image.

Further, the processing device 130 may identify the feature point(s) of the target subject from the second optical image using the feature point recognition model. Compared with identifying of the feature point(s) of the target subject from the first optical image, the identifying of the feature point(s) from the second optical image may involve less computational data, and thereby having an improved efficiency.

In some embodiments, the feature point recognition model may include a deep learning model, such as a DNN model, a CNN model, an RNN model, a FPN model, an Alpha Pose model, or the like, or any combination thereof.

In some embodiments, the feature point recognition model may include a feature extraction network. The feature extraction network may be configured to generate feature maps by extracting image features from the first optical image (or the second optical image) input into the feature point recognition model. In some embodiments, the feature extraction network may include various networks for extracting image features from an image. Exemplary feature extraction networks may include a CNN, a VGG network, a feature extraction network in Alpha Pose model.

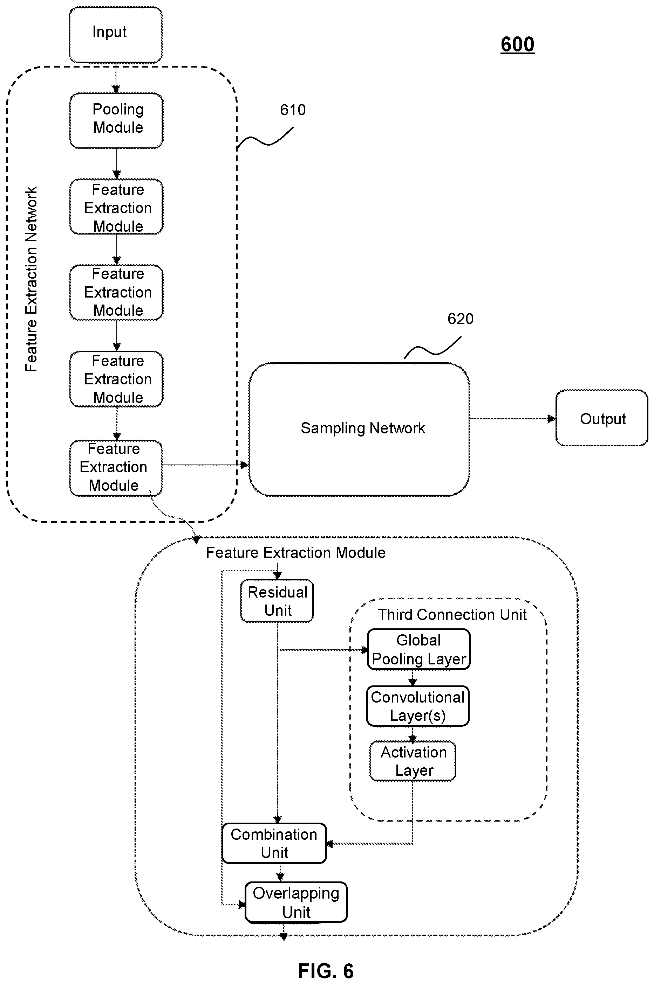

In some embodiments, the feature point recognition model may include a sampling network. The sampling network may be configured to determine information relating to the feature point(s) of the target subject based on the feature maps. For example, the sampling network may perform a sampling operation (e.g., an up-sampling operation) on the feature maps, and output the feature point(s) of the target subject. In some embodiments, the sampling network may be configured to determine a probability that each point in the first optical image is a feature point of the target subject. In some embodiments, the sampling network may include various networks for sampling. Exemplary sampling networks may include a NN, a CNN, a sampling network of an Alpha Pose model, or the like. More descriptions for the feature point recognition model may be found elsewhere in the present disclosure (e.g., FIG. 6 and the descriptions thereof).

In some embodiments, the obtaining of the feature point recognition model may be performed in a similar manner as that of body part boundary recognition model. In some embodiments, the feature point recognition model may be generated according to a machine learning algorithm. Merely by way of example, the feature point recognition model may be trained according to a supervised learning algorithm by the processing device 130 or another computing device (e.g., a computing device of a vendor of the feature point recognition model). The processing device 130 may obtain a plurality of second training samples and a second preliminary model. Each second training sample may include a second sample optical image of a second sample subject and a second training label. The second training label may include one or more sample feature points of the second sample subject in the second sample optical image. In some embodiments, the second training label may include one or more sample points in the second sample optical image, and a sample probability that each sample point in the second sample optical image is a sample feature point of the second sample subject. For example, the second training label may be a sample heatmap indicating a sample probability that each sample point in the second sample optical image is a sample feature point of the second sample subject.

The feature point recognition model may be generated by training the second preliminary model using the plurality of second training samples according to a second total loss function. The second total loss function may be used to measure a discrepancy between a result predicted by the second preliminary model and the corresponding second training table. In some embodiments, the second total loss function may include a fourth loss function or a fifth loss function. The fourth loss function may be used to measure a discrepancy between one or more points in the second sample optical image predicted by the second preliminary model and the sample point(s) in the second sample optical image. The fifth loss function may be used to measure a discrepancy between a probability that each point in the second sample optical image is a feature point of the second sample subject predicted by the second preliminary model and the sample probability that each sample point in the second sample optical image is a sample feature point of the second sample subject. The optimization objective of the training of the second preliminary model may be that the second total loss function satisfies a second preset condition by adjusting parameters of the second preliminary model. For example, the optimization objective of the training of the second preliminary model may be that the value of the second total loss function is smaller than a certain value.

In 330, the processing device 130 (e.g., the identifying module 204) may identify, from the first optical image, an image region corresponding to the target region of the target subject based on the one or more body part boundaries and one or more feature points, wherein at least one first edge of the image region is determined based on the one or more body part boundaries, and at least one second edge of the image region is determined based on the one or more feature points.

As used herein, the image region corresponding to the target region of the target subject refers to a region in the first optical image that includes the target region of the subject. For example, the image region corresponding to the target region of the target subject may be a region in the first optical image that includes the target region and optionally surrounding regions of the target region.

In some embodiments, the processing device 130 may obtain an examination target (e.g., an examination protocol) from the medical system 100 or a user terminal. Further, the processing device 130 may determine information relating to the target region of the subject based on the examination target of the target subject. In some embodiments, the examination target may include the information relating to the target region. Exemplary information relating to the target region may include a body part of the target subject corresponding to the target region, a scan range of the target region, or the like. For example, the examination target for chest examination may indicate that the target region of the target subject needs to be scanned is the chest, and the scan range of the chest is the torso from the neck to the lumbar vertebra. In some embodiments, scan ranges corresponding to different body parts may be generated previously, and stored in a storage device. The processing device 130 may obtain the scan ranges of the target region according to the body part corresponding to the target region from the storage device as a portion of the examination target.

The processing device 130 or a user (e.g., a doctor) may determine at least one target body part boundary from the one or more body part boundaries and at least one target feature point from the one or more feature points based on the information relating to the target region. For example, if the examination target indicates that the target region of the target subject needs to be scanned is the chest, and the scan range of the chest is the torso from the neck to the lumbar vertebra, the processing device 130 may select the at least one body part boundary corresponding to the chest from the one or more body part boundaries as the at least one target body part boundary, and at least one feature point on the neck and the lumbar vertebra from the one or more feature points as the at least one target feature point.

Further, the processing device 130 may determine the image region based on the at least one target body part boundary and the at least one target feature point. In some embodiments, the at least one first edge of the image region may be determined based on the at least one target body part boundary, and the at least one second edge of the image region may be determined based on the at least one target feature point. In some embodiments, the processing device 130 may designate at least one edge of the at least one target body part boundary as the at least one first edge of the image region. The processing device 130 may determine at least one line (e.g., at least one horizontal line) passing through the at least one target feature point as the at least one second edge of the image region. The processing device 130 may determine a region in the first optical image enclosed by the at least one first edge and the at least one second edge as the image region. In some embodiments, the processing device 130 may determine a region enclosed by at least one edge of the at least one target body part boundary as the image region.

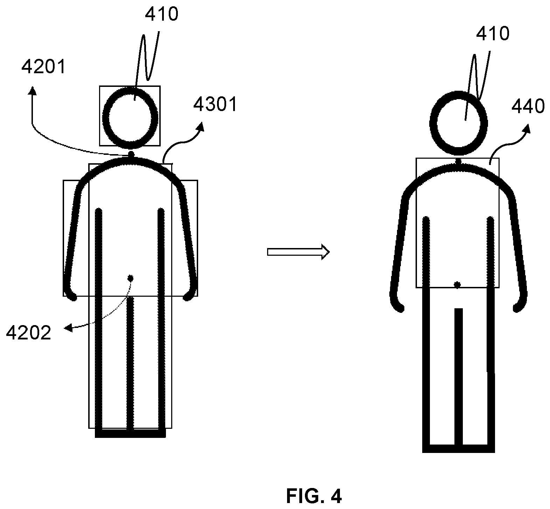

For example, FIG. 4 is a schematic diagram for determining an image region 440 according to some embodiments of the present disclosure. The examination target in FIG. 4 indicates that a target region of a target subject 410 needs to be scanned is the chest, and the scan range of the chest is the torso from the neck to the lumbar vertebra. As shown in the FIG. 4, the processing device 130 may select the bounding box 4301 corresponding to the chest as the target body part boundary of the target subject 410, and feature points 4201 and 4202 on the neck and the lumbar vertebra as the target feature points of the target subject 410. Then, the processing device 130 may determine first edges of image region 440 based on the bounding box 4301. Specifically, the left and right edges of the bounding box 4301 may be designated as the first edges. The processing device 130 may determine horizontal lines passing through the target feature points 4201 and 4202 as the second edges of the image region 440. Accordingly, the image region 440 may be a region enclosed by the first edges and the second edges.

In 340, the processing device 130 (e.g., the determination module 206) may determine, based on the image region, whether a positioning procedure of the medical device can be started.

In some embodiments, in order to obtain medical image data of the target region of the target subject, the positioning procedure of the medical device may be performed before the medical scan. Specifically, one or more components (e.g., a radiation source, a beam limiter, etc.) of the medical device needs to move to their respective target positions by performing the positioning procedure of the medical device. In some embodiments, the positioning procedure of the medical device may be performed based on a positioning target of the medical device. The positioning target of the medical device may include the target positions of one or more components of the medical device.

In some embodiments, the medical device may be a radioactive medical device (e.g., an X-ray scanner). In order to obtain the medical image data of the target region of the target subject, it is necessary to adjust a radiation range of the radiation (i.e., a light field of the medical device) so that the light field of the medical device covers the target region, for example, making the light field coincide with the target region. Accordingly, the positioning target of the radioactive medical device may include a position, a size, etc. of the light field of the radioactive medical device.

In some embodiments, a spatial region corresponding to the image region may be a region to be scanned. The positioning target of the medical device may be determined according to the image region. Specifically, the target positions of one or more components of the medical device, and the position and the size of the light field may be determined according to the image region. For example, the size of the light field may be determined according to a size of the image region, and the target positions of one or more components of the medical device and the position of the light field may be determined according to a position of the image region.

In some embodiments, before the positioning procedure of the medical device can be started, the target region of the target subject needs to substantially remains stationary, that is, the target region of the target subject does not have an obvious motion, so that the medical device may be positioned accurately. In some embodiments, the processing device 130 may obtain a reference optical image captured earlier than the first optical image. For example, the processing device 130 may select one optical image captured earlier than the first optical image from the optical image(s) described in operation 310 as the reference optical image. A difference between an acquisition time of the reference optical image and an acquisition time of the first optical image may be set manually by a user (e.g., an engineer) according to an experience value or a default setting of the medical system 100, or determined by the processing device 130 according to an actual need. In some embodiments, one or more optical images (e.g., 6 optical images, 2 optical images, etc.) may be captured between the reference optical image and the first optical image. Alternatively, the reference optical image and the first optical image may be two consecutively captured optical images.

The processing device 130 may determine a reference image region in the reference optical image corresponding to the target region of the target subject. The processing device 130 may further determine whether the positioning procedure of the medical device can be started based on the reference image region and the image region. In some embodiments, the processing device 130 may determine a displacement of the target subject based on the reference image region and the image region. For example, the processing device 130 may determine a distance between a center of the reference image region and a center of the image region as the displacement of the target subject. The processing device 130 may determine whether the displacement exceeds a displacement threshold. The displacement threshold may be set manually by a user (e.g., an engineer) according to an experience value or an actual need, or a default setting of the medical system 100, such as 7cm, 8cm, etc.

In response to determining that the displacement exceeds the displacement threshold, the processing device 130 may determine that the target subject does not remain stationary. In this case, the target region of the target subject has an obvious motion, and the positioning procedure of the medical device can not be started. The processing device 130 may sequentially obtain an additional optical image later than the first optical image, and repeat operations 310-340 to determine whether the positioning procedure of the medical device can be started.

In response to determining that the displacement does not exceed the displacement threshold, the processing device 130 may determine that the target subject substantially remains stationary. In this case, the position of the target region of the target subject changes little or remains unchanged, and the positioning procedure of the medical device can be started. The processing device 130 may determine the positioning target of the medical device according to the image region or reference image region. Alternatively, the processing device 130 may determine the positioning target of the medical device according to an image region corresponding to the image region of the first optical image in an optical image between the reference optical image and the first optical image. Further, the processing device 130 may perform the positioning procedure of the medical device according to the positioning target of the medical device. In some embodiments, a control instruction may be generated according to the positioning target of the medical device, and the medical device may be controlled to perform the positioning procedure via the control instruction. In some embodiments, a user may control the medical device to perform the positioning procedure according to the positioning target.

As described elsewhere in the present disclosure, conventional medical scan positioning approaches often involves a lot of human intervention, which have low efficiency and accuracy. According to some embodiments of the present disclosure, the processing device 130 may identify the image region corresponding to the target region of the target subject from the first optical image based on the one or more body part boundaries and one or more feature points, and further determine whether the positioning procedure of the medical device can be started based on the image region. In response to determining that the positioning procedure of the medical device can be started, the processing device 130 may determine the positioning target of the medical device according to the image region or reference image region, and perform the positioning procedure of the medical device according to the positioning target of the medical device. Compared with the conventional medical scan positioning approaches, some embodiments of the present disclosure may be implemented with reduced or minimal or without user intervention, which is more efficient and accurate by, e.g., reducing the workload of a user, cross-user variations, and the time needed for the positioning.

Conventionally, a region to be scanned of a subject is determined based only on one or more body part boundaries or one or more feature points of the subject, which has a limited accuracy. According to some embodiments of the present disclosure, the information relating to the target region may be determined quickly and accurately based on the examination target of the target subject, and the at least one target body part boundary and the at least one target feature point may be accurately according to the information relating to the target region. Further, the image region may be determines based on the at least one target body part boundary and the at least one target feature point. Compared with the conventional approach for determining the region to be scanned, some embodiments of the present disclosure may obtain a more accurate region to be scanned (i.e., the image region), thereby improving the accuracy of the positioning of the medical device performed based on the positioning target of the medical device that is determined according to the image region. Moreover, in some embodiments, the determining of the image region may be implemented based on one or more machine learning models such as the body part boundary recognition model, the feature point recognition model, etc. The utilization of the machine learning model(s) may further improve the accuracy and/or efficiency of the determination of the image region.

FIG. 5 is a schematic diagram illustrating an exemplary body part boundary recognition model 500 according to some embodiments of the present disclosure.

In some embodiments, the body part boundary recognition model may include a backbone network 510 and a detection network 520. The backbone network 510 may be configured to generate feature maps by extracting image features from an optical image (e.g., the first optical image described in FIG. 3). The detection network 520 may be configured to determine information associated with at least one body part boundary of a target subject in the optical image based on the feature maps from the backbone network 510. For example, the feature maps from the backbone network 510 may be input into the detection network 520, and the detection network 520 may output one or more bounding boxes of one or more body parts of the target subject and/or information relating to the one or more bounding boxes. In some embodiments, the information relating to the one or more bounding boxes may include classification information of each bounding box, position information of each bounding box, confidence coefficient information corresponding to each bounding box, or the like, or any combination thereof.

In some embodiments, the backbone network 510 may include at least one cross stage partial, CSP, module and a spatial pyramid convolutional, SPC, module connected to the at least one CSP module. The at least one CSP module may be configured to obtain at least one first feature map by extracting image features from the optical image. The SPC module may be configured to obtain a second feature map by extracting image features with different scales from the optical image or the at least one first feature map.

In some embodiments, as shown in FIG. 5, a CSP module may include a first convolutional unit, a first processing unit and a second processing unit connected to an output end of the first convolutional unit, a first connection unit connected to output ends of the first processing unit and the second processing unit, an activation function unit connected to an output end of the first connection unit, and a second convolutional unit connected to an output end of the activation function unit.

In some embodiments, the first convolutional unit or the second convolutional unit may include one or more convolutional layers that are sequentially connected, and sizes of convolution kernels of the convolutional layers may be set according to experience or actual needs.

In some embodiments, the first processing unit may include one or more convolutional layers and an activation function layer connected to output ends of the one or more convolutional layers. In some embodiments, the second processing unit may be the same as or similar to the first processing unit.

In some embodiments, the activation function unit may include a batch normalization, BN, layer and an activation function layer (e.g., a linear rectification function, ReLU) connected to the BN layer.

In some embodiments, the first connecting unit may be used to combine images received by the first connecting unit. Merely by of example, the first connecting unit may be used to combine an output of the first processing unit and an output of the second processing unit. For example, the output of the first processing unit and the output of the second processing unit may be combined in a channel dimension by first connection unit.

The at least one CSP module may solve a problem of large amount of calculation in the data processing process. Specifically, each CSP module may divide an output of the first convention unit into two parts (i.e., an input of the first processing unit and an input of the second processing unit), and then merge the two parts through a cross-stage hierarchy (i.e., the first connecting unit), which may reduce the amount of computation while maintaining the accuracy.

In some embodiments, as shown in FIG. 5, the SPC module may include a third convolutional unit, a plurality of fourth convolutional units, and a second connection unit. In some embodiments, the third convolutional unit may include one or more convolutional layers that are sequentially connected, and sizes of convolution kernels of the convolutional layers may be set according to experience or actual needs, such as 1*1. The plurality of fourth convolutional units may be connected to an output end of the third convolutional unit, and the second connection unit may be connected to output ends of the plurality of fourth convolutional units respectively. Each fourth convolutional unit may be configured to receive an input from the third convolutional unit and transmit an output to the second connection unit. The second connection unit may be configured to combine outputs of the third convolutional unit and outputs of the plurality of fourth convolutional units. For example, the outputs of the third convolutional unit and the outputs of the plurality of fourth convolutional units may be combined in a channel dimension by second connection unit.

In some embodiments, the SPC module may further include a fifth convolutional unit connected to an output end of the second connection unit. The fifth convolutional unit may include one or more sequentially connected convolutional layers, and sizes of convolution kernels of the convolutional layers may be set according to experience or actual needs, such as 1*1.

In some embodiments, each fourth convolutional unit may include one or more sequentially connected convolutional layers. The plurality of fourth convolutional units may have kernels of different sizes. For example, the SPC module may include 3 fourth convolutional units A1, A2, and A3. The fourth convolutional unit A1 may include one or more sequentially connected convolutional layers with a kernel size of 5*5. The fourth convolutional unit A2 may include one or more sequentially connected convolutional layers with a kernel size of 9*9. The fourth convolutional unit A3 may include one or more sequentially connected convolutional layers with a kernel size of 13*13.

By using the SPC module described above, image features of different scales may be extracted through the plurality of fourth convolutional units with kernels of different sizes, and the image features of different scales may be further combined, which may improve the processing effect of the subsequent detection network 520.

In some embodiments, as shown in FIG. 5, two CSP modules in the backbone network 510 may output two first feature maps to the detection network 520, and the SPC module in the backbone network 510 may output one second feature map to the detection network 520. The detection network 520 (e.g., the head network in YOLO model) may process the two first feature maps and the second feature map, and output final feature maps of three scales. Each final feature map may include the bounding boxes of the one or more parts of the target subject and/or information relating to the one or more bounding boxes. The final bounding boxes and/or information relating to the final bounding boxes may be determined based on the final feature maps of three scales in various manners. For example, the final bounding boxes and/or information relating to the final bounding boxes may be determined by decoding information in the final feature maps of three scales.

FIG. 6 is a schematic diagram illustrating an exemplary feature point recognition model 600 according to some embodiments of the present disclosure.

As shown in FIG. 6, the feature point recognition model 600 may include a feature extraction network 610 and a sampling network 620. The feature extraction network 610 may be configured to generate feature maps by extracting image features from an optical image (e.g., the first optical image). The sampling network 620 may be configured to determine information relating to one or more feature points of a target subject based on the feature maps extracted by the feature extraction network 610.

In some embodiments, the feature extraction network 610 may include a pooling module and one or more feature extraction modules connected to an output end of the pooling module.

The pooling module may include a pooling layer such as a maximum pooling layer, an average pooling layer. In some embodiments, each feature extraction module may include a residual unit, a third connection unit, a combination unit, and an overlapping unit. The residual unit may be configured to obtain a residual image based on an input image (e.g., an image output by the pooling module) of the residual unit. The third connection unit may be configured to determine weights each of which corresponds one of feature values of the residual image. The combination unit may be configured to obtain a combination image based on the weights and the feature values. For example, the combination image may be generated by weighting and summing the feature values and their weights. The overlapping unit may be configured to overlap the combination image and the input image of the residual unit to generate an output of the feature extraction module.

In some embodiments, the third connection unit may include a global pooling layer, one or more convolutional layers connected to an output end of the global pooling layer, and an activation layer connected to output end(s) of the convolutional layer(s). The activation layer may be configured to a sigmoid function or other feasible activation functions. Sizes of kernels of the convolutional layer(s) may be set according to experience or requirements.

By using a feature extraction module described above, the weights of the feature values of the residual image may be obtained through the third connection unit. The weight corresponding to each feature value may be used to evaluate the importance of the feature value. Moreover, weight corresponding to each feature value may be updated by iteratively updating the parameters of the convolutional layer(s) in the third connection unit during the training of the feature point recognition model 600. For example, the weight of the feature channel that is important for the current training task may be increased and the weight of feature channel that is not important for the current training task may be reduced. Then the combination image may be obtained based on the weights and the feature values. In this way, the important feature values of the residual image may be assigned with relatively great weights and the unimportant feature values of the residual image may be assigned with relatively small weights, which may improve the accuracy of the combination image and the output of the feature extraction module determined based on the combination image.

In some embodiments, is the medical device is a radioactive medical device (e.g., a CT scanner, an RT device), during the process of the scan or treatment of the target subject, some rays or ray beams may radiate to one or more medical workers who participate in the scan or the treatment, which results in potential radiation risks faced by the medical worker(s) during multiple scans or treatments. Therefore, it is desirable to provide methods and systems for determining a radiation region of a radioactive medical device for prompting medical workers to stay away from the radiation region.

FIG. 7 is a flowchart illustrating an exemplary process 700 for determining a radiation region of a radioactive medical device according to some embodiments of the present disclosure.

In 710, during a scan or a treatment performed by the radioactive medical device on the target subject, the processing device 130 (e.g., the acquisition module 202) may obtain a second optical image indicating the scene of the scan or the treatment.

The second optical image may include one or more medical workers who participate in the scan or the treatment, the radioactive medical device, and the target subject. The medical workers may include a radiologist who performs the scan, a doctor who performs the treatment or medical diagnosis (e.g., a surgeon who performs a surgery guided by the scan), an assistant medical worker, an anesthesiologist, etc. For illustration purposes, the following descriptions are provided with reference to a scan of the target subject.

In some embodiments, the second optical image may be captured by an image acquisition device (e.g., the image acquisition device 120). In some embodiments, the processing device 130 may obtain a 2D optical image captured by a 2D image acquisition device as the second optical image. In some embodiments, the processing device 130 may obtain a 3D optical image captured by a 3D image acquisition device as the second optical image. Specifically, the 3D image acquisition device may accurately detect a distance between each point in the scene of the scan and the 3D image acquisition device, so as to obtain 3D spatial coordinates of each point in the scene of the scan, and then obtain the 3D optical image by modeling based on the 3D spatial coordinates. In some embodiments, the processing device 130 may obtain a plurality of 2D optical images captured by a plurality of image acquisition devices, and then generate the 3D optical image by performing a 3D reconstruction on the plurality of 2D optical images.

In 720, the processing device 130 (e.g., the determination module 206) may determine, based on the second optical image, first information of the one or more medical workers and second position information of the radiation region of the radioactive medical device.

In some embodiments, the first information of each medical worker may include first position information of the medical worker, such as a first position of the medical worker a confidence coefficient of the first position, etc. In some embodiments, the first information of each medical worker may include a classification of the medical worker.

In some embodiments, the first position of the medical worker may be defined by coordinates of the medical worker. For example, the coordinates of the medical worker may be represented by coordinates in a rectangular coordinate system established with a point in the second optical image as the origin. As another example, the coordinates of the medical worker may be represented by coordinates in a rectangular coordinate system or a three-dimensional coordinate system established with a point in the scene of the scan or the treatment as the origin. In some embodiments, the coordinates of the medical worker may be represented by a set of coordinate points on the medical worker (e.g., a set of coordinate points on a boundary of the medical worker), or coordinates of a center point of the medical worker. In some embodiments, the coordinates of the medical worker may be represented by a set of coordinate points of a bounding box (e.g., a rectangular bounding box) enclosing the medical worker. For example, as shown in FIG. 8, the coordinates of a medical worker 840 may be represented by a set of coordinate points on a rectangular bounding box A (e.g., coordinates of the right edge of the rectangular bounding box A) or coordinates of the center point of the rectangular bounding box A.

In some embodiments, medical workers may be classified according to their responsibilities. For example, the classification of the medical worker may include a doctor, a nurse, a radiologist, an assistant medical worker, an anesthesiologist, or the like, or any combination thereof. In some embodiments, the confidence coefficient of the first position may indicate a probability that there is a medical worker at the first position (e.g., a probability that there is a medical worker in the rectangular bounding box A in FIG. 8). The higher the confidence coefficient of the first position is, the greater probability that there is a medical worker at the first position may be.

In some embodiments, the processing device 130 may determine the first information of the one or more medical workers based on the second optical image. For example, the processing device 130 may determine the first information of the one or more medical workers using an image recognition technology based on the second optical image.

In some embodiments, the processing device 130 may determine the first information of the one or more medical workers based on the second optical image using a position information determination model. The position information determination model may be a model for identifying one or more subjects in an image such as the one or more medical workers, the target subject, one or more components of the radioactive medical device, to determine information relating to the one or more subjects. In some embodiments, different subjects may be identified using different position information determination models.

Merely by way of example, the second optical image may be input into the position information determination model, and the position information determination model may output the first information. In some embodiments, the first information output by the position information determination model may be marked on the second optical image with markers such as a marking box or a marking point, for the convenience of user observation. In some embodiments, the position information determination model may include a deep learning model. In some embodiments, the deep learning model may include a Yolo deep learning model, a single shot multibox detector, SSD, model, a spatial pyramid pooling networks, SSP-Net, model, a Region-CNN, R-CNN, model, a Fast R-CNN model, a Faster R-CNN model, a region-based fully convolutional networks, R-FCN, model, etc.

In some embodiments, the obtaining of the position information determination model may be performed in a similar manner as that of body part boundary recognition model as described in connection with operation 320. In some embodiments, the position information determination model may be generated according to a machine learning algorithm. More descriptions regarding the position information determination model may be found elsewhere in the present disclosure (e.g., FIG. 9 and the descriptions thereof).

As used herein, the radiation region refers to an area where a radiation dose is greater than a dose threshold. In some embodiments, the radiation region may be a regular or irregular shape such as a sphere or circular area. If the second optical image is a 3D optical image, the radiation region may be a sphere. If the second optical image is a 2D optical image, the radiation region may be a circular area.

Merely by way of example, FIG. 8 is a schematic diagram illustrating an exemplary second optical image according to some embodiments of the present disclosure. As shown in FIG. 8, a tube 810 of a C-arm device may emit X-rays to a target object 850, and the X-rays may be received by a detector 830 after passing through the target object 850 and a scanning table 820. When the X-rays passes through the target object 850 and the scanning table 820, a portion of the X-rays may be scattered to generate scattered rays. The scattered rays and the X-rays may generate a radiation field in an area centered on the target object 850. In an area covered by the radiation field, a portion of the area where the radiation dose exceeds the dose threshold may be determined as the radiation region. For example, a partial region on the right side of the arc C in FIG. 8 may have the radiation dose exceeding the dose threshold, and therefore the region on the right side of the arc C be determined as the radiation region.

In some embodiments, the second position information of the radiation region may include a set of coordinates in the radiation region, a radiation dose of each coordinate in the radiation region. In some embodiments, the second position information of the radiation region may be represented by a set of coordinate points on a boundary of the radiation region or a portion of the boundary (e.g., a portion of the boundary adjacent to the medical workers). In some embodiments, the second position information may include a radiation distance of the radioactive medical device. In some embodiments, the radiation distance of the radioactive medical device may include a distance between the target region of the target subject and a boundary of the radiation region. For example, the radiation distance of the radioactive medical device may include a maximum distance between the target region of the target subject and the boundary of the radiation region. As another example, the radiation distance of the radioactive medical device may include a plurality of distances between a central point of the target region of the target subject and a plurality of points on the boundary of the radiation region.

In some embodiments, the processing device 130 may obtain first radiation information associated with radiation parameters of the radioactive medical device. Exemplary radiation parameters may include a tube voltage, a tube current, etc., of a tube for emitting radiation rays, an effective time of a pulse, a radiation dose of the radiation rays, an incident area of the radiation rays, or the like, or any combination thereof.

The processing device 130 may determine, based on the second optical image, second radiation information using the position information determination model. Merely by way of example, the second optical image may be input into the position information determination model, and the position information determination model may output the second radiation information. In some embodiments, the second radiation information output by the position information determination model may be marked on the second optical image with markers such as a marking box or a marking point, for the convenience of user observation.

In some embodiments, the second radiation information may include position information associated with the target subject. Exemplary position information associated with the target subject may include target position information, body thickness information, etc., of the target subject. In some embodiments, the target position information of the target subject may include a target position of the target subject. In some embodiments, the target position of the target subject may be represented by a set of coordinates of multiple points in a scanned region (e.g., the image region described in operation 340) of the scan of the target subject or coordinates of a center point of the scanned region. For example, the target position of the target subject 850 in FIG. 8 may be represented by a set of coordinates of multiple points on the rectangular bounding box B or coordinates of the center point B₀ of rectangular bounding box B. In some embodiments, the body thickness information of the target subject may include a thickness (e.g., a height of the rectangular bounding box B in FIG. 8) of the target region of the target subject.

In some embodiments, the second radiation information may further include position information associated with the radioactive medical device. Exemplary position information associated with the radioactive medical device may include position information of the tube and a detector of the radioactive medical device. In some embodiments, the position information of the tube may be represented by a set of coordinates of multiple points on the tube or coordinates of a center point of the tube. In some embodiments, the position information of the detector may be represented by a set of coordinates of multiple points on the detector or coordinates of a center point of the detector.

In some embodiments, the processing device 130 may obtain the first radiation information or the second radiation information from one or more components of the medical system 100 or an external source via a network.

Further, the processing device 130 may determine the second position information of the radiation region of the radioactive medical device based on the first radiation information and the second radiation information. In some embodiments, the processing device 130 may obtain a relationship between radiation information and radiation distances. The relationship between radiation information and radiation distances may indicate a mapping relationship between the radiation parameters (i.e., the first radiation information), the position of the target subject (i.e., the second radiation information), and the radiation distances. In some embodiments, the radiation distance of the radioactive imaging device may include one or more distances between the position of the target subject and the boundary of the radiation region. For example, as shown in FIG. 8, the radiation distance may include the distance between the center point B₀ of the target region of the target subject 850 and each point on the arc C of the boundary of the radiation region. In some embodiments, in order to improve data processing efficiency, the radiation distance may be one of the distances between the target subject and the boundary of the radiation region, for example, the distance d between the center point B₀ of the target region and the arc C in the horizontal direction as shown in FIG. 8.

In some embodiments, the relationship between radiation information and radiation distances may be represented by various forms. For example, the relationship between radiation information and radiation distances may be represented by a relationship table. In some embodiments, the radiation information in the relationship table may include one or more parameters of the first radiation information and the second radiation information. In some embodiments, the radiation information in the relationship table may further include imaging parameters of the image acquisition device, such as a shooting angle, a focal length, a field of view, an aperture, etc.

In some embodiments, the processing device 130 may determine the relationship between radiation information and radiation distances based on multiple reference scans of reference subjects. The multiple reference scans may correspond to different scan conditions. The scan condition may be specified by imaging parameters of the image acquisition device and scan parameters (e.g., radiation doses) of the radioactive medical device. During each reference scan, radiation doses at different distances from the reference object may be measured using a radiation measurement device. A reference radiation region may be determined according to the radiation doses at different distances from the reference object. Then, reference radiation information of the reference radiation region and one or more reference radiation distances between the reference subject and one or more reference points on a boundary of the reference radiation region (e.g., a longest distance between a central point of the reference subject and the boundary) may be determined based on the reference radiation region. The processing device 130 may determine the relationship between radiation information and the radiation distances based on the one or more reference radiation distance(s) and the reference radiation information obtained by each reference scan. For example, the processing device 130 may establish a relationship table between radiation information and radiation distances according to the one or more reference radiation distance(s) and the reference radiation information obtained by each reference scan. In some embodiments, the processing device 130 may perform an interpolation operation on the reference radiation distances and the reference radiation information obtained by the multiple reference scans to obtain a complete relationship table.

The processing device 130 may determine the second position information of the radiation region of the radioactive medical device based on the relationship between radiation information and radiation distances, the first radiation information, and the second radiation information. For example, the processing device 130 may determine the radiation distance of the radioactive imaging device according to the first radiation information and the second radiation information from the relationship table between radiation information and radiation distances. The processing device 130 may determine the second position information of the radiation region based on the radiation distance. For example, the processing device 130 may determine the boundary of the radiation region according to the radiation distance and the target position of the target subject.

In some embodiments, the processing device 130 may determine the second position information of the radiation region using a radiation region information determination model based on the first radiation information and the second radiation information. Merely by way of example, the first radiation information and the second radiation information may be input into the radiation region information determination model, and the radiation region information determination model may output the second position information of the radiation region. In some embodiments, the second position information of the radiation region (e.g., the radiation distance, the boundary, etc., of the radiation region) output by the radiation region information determination model may be marked on the second optical image with markers such as a marking box or a marking point, for the convenience of user observation. In some embodiments, the radiation region information determination model may include a convolutional recurrent neural network, CRNN, a CNN, a deep convolutional neural network, DCNN, a RNN, or the like, or any combination thereof.

In some embodiments, the obtaining of the radiation region information determination model may be performed in a similar manner as that of body part boundary recognition model as described in connection with operation 320. In some embodiments, the radiation region information determination model may be generated according to a machine learning algorithm. More descriptions for the radiation region information determination model may be found elsewhere in the present disclosure (e.g., FIG. 9 and the descriptions thereof).

According to some embodiments of the present disclosure, the second position information of the radiation region may be determined using one or more machine learning models. Specifically, the second radiation information may be determined using the position information determination model. Further, the second position information of the radiation region may be determined based on the relationship between radiation information and radiation distances, the first radiation information, and the second radiation information. Alternatively, the second position information of the radiation region may be determined using the radiation region information determination model based on the first radiation information and the second radiation information. Since each machine learning model may learn the optimal mechanism for obtaining corresponding results based on a large amount of data, the second position information of the radiation region determined using the one or more machine learning models may be relatively more accurate.

In 730, the processing device 130 (e.g., the determination module 206) may determine, based on the first information and the second position information, whether at least one of the one or more medical workers need to change positions.

In some embodiments, for each of the one or more medical workers, the processing device 130 may determine a distance between the medical worker and the target subject based on the first position information corresponding to the medical worker and the target position information of the target subject. For example, the processing device 130 may determine a distance between the first position of the medical worker and the target position of the target subject based on the first position information corresponding to the medical worker and the target position information as the distance between the medical worker and the target subject. Merely by way of example, the processing device 130 may determine a distance between the bounding box for representing the first position of the medical worker described in operation 720 and the center point of the scanned region. For example, as shown in FIG. 8, the processing device 130 may determine a distance D between the right edge of the rectangular bounding box A and the center point B₀ of the rectangular bounding box B, and designate the distance D as the distance between the medical worker 840 and the target subject 850.

In response to determining that the distance between the medical worker and the target subject is smaller than or equal to the radiation distance of the radioactive medical device, the processing device 130 may determine that the medical worker is located in the radiation region. In response to determining that the distance between the medical worker and the target subject is greater than the radiation distance of the radioactive medical device, the processing device 130 may determine that the medical worker is not located in the radiation region. In response to determining that the distance between the medical worker and the target subject is greater than the radiation distance of the radioactive medical device by a distance threshold (e.g., 1 cm, 5cm, 10 cm, etc.), the processing device 130 may determine that the medical worker is close to the radiation region.

In response to determining that the medical worker is located in or close to the radiation region, the processing device 130 may determine that the medical worker need to change his/her position. In these occasions, the processing device 130 may prompt the medical worker in various manners, for example, generating a voice broadcast, an indicator light flashing, an alarm sound, etc. For example, the processing device 130 may directly cause an output device of a terminal to prompt the medical worker. As another example, the processing device 130 may send a prompt instruction to a terminal to cause an output device of the terminal to prompt the medical worker.

According to some embodiments of the present disclosure, the first information of the one or more medical workers and the second position information of the radiation region may be determined accurately and efficiently based on the second optical image using one or more machine learning models (e.g., the position information determination model, the radiation region information determination model), which may achieve the automatic and real-time monitoring of positions of the one or more medical workers. Further, whether the medical worker is located in or close to the radiation region may be accurately determined based on the accurate first information and second position information, so that the medical worker(s) can be monitored to stay away from the radiation region, thereby protecting the medical worker(s) from radiation damage.

In some embodiments, the position information determination model or the radiation region information determination model may be generated by performing process 900 as shown in FIG. 9. FIG. 9 is a flowchart illustrating an exemplary process 900 for generating a position information determination model or a radiation region information determination model according to some embodiments of the present disclosure.

In 910, the processing device 120 (e.g., the model generation module 208) may obtain a plurality of third training samples.

In some embodiments, the plurality of third training samples may be used for generating the position information determination model, which can be used to determine the first information of the one or more medical workers. In this case, each third training sample may include a sample optical image including a sample medical worker and sample information of the sample medical worker. The sample information of the sample medical worker may be used to determine a training label for model training (i.e., the ground truth information of the sample medical worker). In some embodiments, the sample information of the sample medical worker may be similar to the first information of the medical worker described in operation 720. The sample information of the sample medical worker may include sample first position information of the sample medical worker, such as a sample first position of the sample medical worker a sample confidence coefficient of the sample first position, etc. In some embodiments, the sample information of sample medical worker may include a sample classification of the sample medical worker.

In some embodiments, the plurality of third training samples may be used for generating the position information determination model, which can be used to determine the second radiation information of the radioactive medical device. In this case, each third training sample may include a sample optical image including a sample radioactive medical device and sample second radiation information of the sample radioactive medical device. The sample second radiation information may be used to determine a training label for model training (i.e., the ground truth sample second radiation information of the sample radioactive medical device). In some embodiments, the sample second radiation information may be similar to the second radiation information described in operation 720. The sample second radiation information may include sample position information associated with a sample target subject being scanned (e.g., sample target position information, sample body thickness information, etc., of the sample target subject), and/or sample position information associated with the sample radioactive medical device (e.g., sample position information of a tube and a detector of the sample radioactive medical device).

In some embodiments, the plurality of third training samples may be used for generating the position information determination model, which can be used to determine both the first information of the one or more medical workers and the second radiation information of the radioactive medical device. In this case, each third training sample may include a sample optical image including both a sample medical worker and a sample radioactive medical device, sample information of the sample medical worker, and sample second radiation information of the sample radioactive medical device. The sample information of the sample medical worker and the sample second radiation information may be used to determine a training label of the model training.

In some embodiments, the plurality of third training samples may be used for generating the radiation region information determination model, which can be determine the second position information of the radiation region. In this case, each third training sample may include sample radiation information and sample second position information of a sample radiation region of a sample radioactive medical device. In some embodiments, the sample radiation information may include sample first radiation information associated with radiation parameters of the sample radioactive medical device and sample second radiation information. The sample second radiation information may be similar to or the same as the sample second radiation information described above. In some embodiments, the sample second position information of the sample radiation region may be similar to the second position information of the radiation region described in operation 720. For example, the sample second position information of the sample radiation region may include a sample radiation distance of the same radioactive medical device.

In some embodiments, the processing device 130 may perform a preprocessing operation on the plurality of third training samples. Exemplary preprocessing operations may include a format conversion, a normalization, an identification, etc.

In some embodiments, the plurality of third training samples may be identified manually by a user or automatically by a processing device.

In some embodiments, the processing device 130 may obtain a training sample (or a portion thereof) from one or more components (e.g., the storage device) of the medical system 100 or an external source (e.g., a database of a third-party) via a network.

In 920, the processing device 130 (e.g., the model generation module 208) may generate the position information determination model or the radiation region information determination model by training a third preliminary model using the plurality of third training samples.

The third preliminary model refers to a model to be trained. The third preliminary model may be of any type of model as described in FIG. 7. In some embodiments, the third preliminary model may include a YoloV3 model, a deep belief network model, a VGG model, an OverFeat model, an R-CNN model, a spatial pyramid pooling network, SSP-Net, model, a Fast R-CNN model, a Faster RCNN model, a R-FCN model, a deeply supervised object detectors, DSOD, model, etc. In some embodiments, the third preliminary model may include a CRNN, a CNN, a DCNN, a RNN or a long/short term memory, LSTM, model, etc.

In some embodiments, the third preliminary model may be trained using the plurality of third training samples according to a loss function. Merely by way of example, the position information determination model for determining the first information of the one or more medical workers may be generated by training the third preliminary model using the plurality of third training samples according to a third total loss function. The third total loss function may be used to measure a discrepancy between a result predicted by the third preliminary model and the corresponding training label (i.e., the ground truth information of the sample medical worker). In some embodiments, the third total loss function may include one or more of a sixth loss function, a seventh loss function, and an eighth loss function. The sixth loss function may be configured to evaluate a predicted result associated with the sample first position of the sample medical worker. Specifically, the sixth loss function may be used to measure a discrepancy between a first position of the sample medical worker predicted by the third preliminary model and the sample first position of the sample medical worker. The seventh loss function may be configured to evaluate a predicted result associated with the sample confidence coefficient of the sample first position. Specifically, the seventh loss function may be used to measure a discrepancy between a confidence coefficient of the sample first position predicted by the third preliminary model and the sample confidence coefficient of the sample first position. The eighth loss function may be configured to evaluate a predicted result associated with the sample classification of the sample medical worker. Specifically, the eighth loss function may be used to measure a discrepancy between a classification of the sample medical worker predicted by the third preliminary model and the sample classification of the sample medical worker.

In some embodiments, the training of the third preliminary model may include the following operations. In operation (1), the plurality of third training samples may be divided into a training set, a validation set, and a test set randomly or according to a preset division rule. For example, 85% of the third training samples may be used as the training set, 10% of the third training samples may be used as the validation set, and 5% of the third training samples may be used as the test set. In operation (2), each third training sample in the training set may be input into the third preliminary model. When the training of the third preliminary model satisfies a termination condition, the training may be stopped, and the third preliminary model with updated parameter values may be designated as a trained machine learning model (i.e., the position information determination model or the radiation region information determination model). Exemplary termination conditions may include a certain count of training iterations has been performed, or the value of the loss function is less than a preset value, etc. In operation (3), each third training sample in the verification set may be input into the trained machine learning model to obtain a predicted verification result output by the trained machine learning model. In operation (4), each predicted verification result output by the trained machine learning model may be compared with the corresponding training label to obtain a comparison result. In some embodiments, the comparison result may include that the predicted verification result matches the training label, or the predicted verification result does not match the training label. For example, if a difference between the predicted verification result and the training label is within a threshold (e.g., 2%), the predicted verification result may be deemed as matching the training label. If the difference between the predicted verification result and the training label is not within the threshold, the predicted verification result may be deemed as mismatching the training label. If the comparison result satisfies a verification requirement, then the operation (5) may be performed. The verification requirement may be set according to actual needs. For example, the verification requirement may be that the predicted verification results obtained based on more than 95% of third training samples in the verification set can match the corresponding training labels. If the comparison result does not satisfy the verification requirement, it is determined that the validation of the trained machine model does not satisfy requirements. For example, the trained machine model may have a low prediction accuracy. The parameters of the trained machine model may be adjusted, and operation (2) may be performed again based on the adjusted parameters. In operation (5), each third training sample in the test set may be input into the trained machine learning model to obtain a predicted teat result. In operation (6), each predicted teat result may be compared with the corresponding training label to determine whether the training satisfies a test requirement. The test requirement may be set according to actual needs. For example, the test requirement may be that the predicted teat results obtained based on more than 98% of third training samples in the test set can match the corresponding training labels. If the training does not satisfy the test requirement, the third preliminary model may be retrained using a plurality of additional third training samples or, the plurality of third training samples may be divided into a new training set, a new validation set, and a new test set to retrain the third preliminary model.

Moreover, certain terminology has been used to describe embodiments of the present disclosure. For example, the terms "one embodiment," "an embodiment," and "some embodiments" mean that a particular feature, structure or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present disclosure.

Further, it will be appreciated by one skilled in the art, aspects of the present disclosure may be illustrated and described herein in any of a number of patentable classes or context including any new and useful process, machine, manufacture, or composition of matter, or any new and useful improvement thereof. Accordingly, aspects of the present disclosure may be implemented entirely hardware, entirely software (including firmware, resident software, micro-code, etc.) or combining software and hardware implementation that may all generally be referred to herein as a "module," "unit," "component," "device," or "system." Furthermore, aspects of the present disclosure may take the form of a computer program product embodied in one or more computer readable media having computer readable program code embodied thereon.

A computer readable signal medium may include a propagated data signal with computer readable program code embodied therein, for example, in baseband or as part of a carrier wave.

Computer program code for carrying out operations for aspects of the present disclosure may be written in any combination of one or more programming languages.

Furthermore, the recited order of processing elements or sequences, or the use of numbers, letters, or other designations therefore, is not intended to limit the claimed processes and methods to any order except as may be specified in the claims.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claim subject matter lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. In some embodiments, a classification condition used in classification or determination is provided for illustration purposes and modified according to different situations.

## Claims

1. A system, comprising:
at least one storage device including a set of instructions for medical imaging; and
at least one processor in communication with the at least one storage device, wherein when executing the set of instructions, the at least one processor is configured to direct the system to perform operations including:
obtaining a first optical image of a target subject that includes a target region to be scanned or treated by a medical device, the first optical image being captured by an image acquisition device before a scan or a treatment of the target region;
identifying, from the first optical image, at least one body part boundary and at least one feature point of the target subject using at least one target recognition model, each of the at least one feature point referring to a point in the first optical image that represents a point of interest or a critical point of the target subject;
identifying, from the first optical image, an image region corresponding to the target region of the target subject based on the at least one body part boundary and the at least one feature point, wherein at least one first edge of the image region is determined based on the at least one body part boundary, and at least one second edge of the image region is determined based on the at least one feature point; and
determining, based on the image region, whether a positioning procedure of the medical device can be started.

2. The system of claim 1, wherein the at least one target recognition model includes a body part boundary recognition model, and
the identifying, from the first optical image, at least one body part boundary and at least one feature point of the target subject using at least one target recognition model includes:
identifying, from the first optical image, the at least one body part boundary of the target subject using the body part boundary recognition model.

3. The system of claim 2, wherein the body part boundary recognition model includes:
a backbone network configured to generate feature maps by extracting image features from the first optical image; and
a detection network configured to determine information associated with the at least one body part boundary based on the feature maps.

4. The system of claim 2 or claim 3, wherein
the body part boundary recognition model is generated by training a first preliminary model using a plurality of first training samples,
each of the plurality of first training samples have a first training label including sample position information, sample confidence coefficient information, and sample classification information relating to a sample body part boundary, and
a loss function for training the first preliminary model include at least one of:
a first loss function configured to evaluate a predicted result associated with the sample position information,
a second loss function configured to evaluate a predicted result associated with the sample confidence coefficient information, or
a third loss function configured to evaluate a predicted result associated with the sample classification information.

5. The system of claim 1, wherein the at least one target recognition model includes a feature point recognition model, and
the identifying, from the first optical image, at least one body part boundary and at least one feature point of the target subject using at least one target recognition model includes:
identifying, from the first optical image, the at least one feature point of the target subject using the feature point recognition model.

6. The system of claim 5, wherein the identifying, from the first optical image, the at least one feature point of the target subject using the feature point recognition model includes:
determining, based on the at least one body part boundary and the first optical image, a second optical image including at least one body part of the target subject;
identifying, from the second optical image, the at least one feature point of the target subject using the feature point recognition model.

7. The system of claim 5, wherein the feature point recognition model includes:
a feature extraction network configured to generate feature maps by extracting image features from the first optical image input into the feature point recognition model; and
a sampling network configured to determine information relating to the at least one feature point of the target subject based on the feature maps.

8. The system of any one of claims 5-7, wherein identifying, from the first optical image, the at least one feature point of the target subject using the feature point recognition model comprises:
obtaining, by inputting the first optical image into the feature point recognition model, a heatmap indicating a probability that each point in the first optical image is a feature point of the target subject; and
determining, based on the heatmap, the at least one feature point of the target subject.

9. The system of any one of claims 1-8, wherein identifying, from the first optical image, an image region corresponding to the target region based on the at least one body part boundary and the at least one feature point includes:
determining information relating to the target region of the subject based on an examination target of the target subject, the information relating to the target region including at least one of a body part of the target subject corresponding to the target region or a scan range of the target region;
determining, based on the information relating to the target region, at least one target body part boundary from the at least one body part boundary and at least one target feature point from the at least one feature point; and
determining, based on the at least one target body part boundary and the at least one target feature point, the image region, wherein the at least one first edge of the image region is determined based on the at least one target body part boundary, and the at least one second edge of the image region is determined based on the at least one target feature point.

10. The system of any one of claims 1-9, wherein the determining, based on the image region, whether a positioning procedure of the medical device can be startedincludes:
obtaining a reference optical image captured earlier than the first optical image;
determining a reference image region in the reference optical image corresponding to the target region of the target subject; and
determining, based on the reference image region and the image region, whether the positioning procedure of the medical device can be started.

11. The system of claim 1, wherein the at least one target recognition model includes body part boundary recognition models corresponding to different body part boundaries of the target subject and/or different feature point recognition models corresponding to feature points that are located on different body parts.

12. The system of claim 3, wherein the backbone network includes at least one cross stage partial, CSP, module and a spatial pyramid convolutional, SPC, module connected to the at least one CSP module,
the at least one CSP module is configured to obtain at least one first feature map by extracting image features from the first optical image; and
the SPC module is configured to obtain a second feature map by extracting image features with different scales from the first optical image or the at least one first feature map.

13. The system of claim 1, wherein each of the at least one body part boundary is represented by a bounding box, and
information relating to the bounding box includes confidence coefficient information corresponding to the bounding box, the confidence coefficient information corresponding to the bounding box including a confidence coefficient indicating a reliability that the region of the first optical image enclosed by the bounding box is a foreground region or a background region.

14. A method, the method being implemented on a computing device having at least one storage device and at least one processor, the method comprising:
obtaining a first optical image of a target subject that includes a target region to be scanned or treated by a medical device, the first optical image being captured by an image acquisition device before a scan or a treatment of the target region;
identifying, from the first optical image, at least one body part boundary and at least one feature point of the target subject using at least one target recognition model, each of the at least one feature point referring to a point in the first optical image that represents a point of interest or a critical point of the target subject; and
identifying, from the first optical image, an image region corresponding to the target region of the target subject based on the at least one body part boundary and the at least one feature point, wherein at least one first edge of the image region is determined based on the at least one body part boundary, and at least one second edge of the image region is determined based on the at least one feature point; and
determining, based on the image region, whether a positioning procedure of the medical device can be started.

15. A system, comprising:
an acquisition module, configured to obtain a first optical image of a target subject that includes a target region to be scanned or treated by a medical device, the first optical image being captured by an image acquisition device before a scan or a treatment of the target region; and
an identifying module, configured to:
identify, from the first optical image, at least one body part boundary and at least one feature point of the target subject using at least one target recognition model, each of the at least one feature point referring to a point in the first optical image that represents a point of interest or a critical point of the target subject;
identify, from the first optical image, an image region corresponding to the target region of the target subject based on the at least one body part boundary and the at least one feature point, wherein at least one first edge of the image region is determined based on the at least one body part boundary, and at least one second edge of the image region is determined based on the at least one feature point; and
determine, based on the image region, whether a positioning procedure of the medical device can be started.

## Patentansprüche

1. System, umfassend:
mindestens eine Speichervorrichtung, die einen Satz Anweisungen für medizinische Bildgebung beinhaltet; und
mindestens einen Prozessor, in Kommunikation mit der mindestens einen Speichervorrichtung, wobei der mindestens eine Prozessor, wenn er den Satz Anweisungen ausführt, konfiguriert ist, um das System anzuweisen, Operationen durchzuführen, die beinhalten:
Erhalten eines ersten optischen Bildes eines Zielsubjekts, das einen Zielbereich beinhaltet, der von einer medizinischen Vorrichtung zu scannen oder zu behandeln ist, wobei das erste optische Bild vor einem Scan oder einer Behandlung des Zielbereichs von einer Bilderfassungsvorrichtung aufgenommen wird;
Identifizieren, anhand des ersten optischen Bildes, mindestens einer Körperteilgrenze und mindestens eines Merkmalspunktes des Zielsubjekts unter Verwendung mindestens eines Zielerkennungsmodells, wobei jeder des mindestens einen Merkmalspunktes auf einen Punkt im ersten optischen Bild verweist, der einen Punkt von Interesse oder einen kritischen Punkt des Zielsubjekts darstellt;
Identifizieren, anhand des ersten optischen Bildes, eines Bildbereichs, der dem Zielbereich des Zielsubjekts entspricht, basierend auf der mindestens einen Körperteilgrenze und dem mindestens einen Merkmalspunkt, wobei mindestens eine erste Kante des Bildbereichs basierend auf der mindestens einen Körperteilgrenze bestimmt wird, und mindestens eine zweite Kante des Bildbereichs basierend auf dem mindestens einen Merkmalspunkt bestimmt wird; und
Bestimmen, basierend auf dem Bildbereich, ob ein Positionierungsvorgang der medizinischen Vorrichtung gestartet werden kann.

2. System nach Anspruch 1, wobei das mindestens eine Zielerkennungsmodell ein Körperteilgrenzen-Erkennungsmodell beinhaltet, und
das Identifizieren, anhand des ersten optischen Bildes, mindestens einer Körperteilgrenze und mindestens eines Merkmalspunktes des Zielsubjekts unter Verwendung mindestens eines Zielerkennungsmodells beinhaltet:
Identifizieren, anhand des ersten optischen Bildes, der mindestens einen Körperteilgrenze des Zielsubjekts unter Verwendung des Körperteilgrenzen-Erkennungsmodells.

3. System nach Anspruch 2, wobei das Körperteilgrenzen-Erkennungsmodell beinhaltet:
ein Backbone-Netzwerk, das konfiguriert ist, um Merkmalskarten durch Extrahieren von Bildmerkmalen aus dem ersten optischen Bild zu generieren; und
ein Detektionsnetzwerk, das konfiguriert ist, um basierend auf den Merkmalskarten Informationen zu bestimmen, die der mindestens einen Körperteilgrenze zugeordnet sind.

4. System nach Anspruch 2 oder Anspruch 3, wobei
das Körperteilgrenzen-Erkennungsmodell durch Trainieren eines ersten vorläufigen Modells unter Verwendung einer Vielzahl von ersten Trainingsproben generiert wird, wobei jede der Vielzahl von ersten Trainingsproben eine erste Trainingsmarkierung aufweist, die Probenpositionsinformationen, Probenkonfidenzkoeffizienteninformationen und Probenklassifizierungsinformationen beinhaltet, die sich auf eine Probenkörperteilgrenze beziehen, und
eine Verlustfunktion zum Trainieren des ersten vorläufigen Modells mindestens eines beinhaltet von:
einer ersten Verlustfunktion, die konfiguriert ist, um ein vorhergesagtes Ergebnis, das den Probenpositionsinformationen zugeordnet ist, zu bewerten,
einer zweiten Verlustfunktion, die konfiguriert ist, um ein vorhergesagtes Ergebnis, das den Probenkonfidenzkoeffizienteninformationen zugeordnet ist, zu bewerten, oder
einer dritten Verlustfunktion, die konfiguriert ist, um ein vorhergesagtes Ergebnis, das den Probenklassifizierungsinformationen zugeordnet ist, zu bewerten.

5. System nach Anspruch 1, wobei das mindestens eine Zielerkennungsmodell ein Merkmalspunkt-Erkennungsmodell beinhaltet, und
das Identifizieren, anhand des ersten optischen Bildes, mindestens einer Körperteilgrenze und mindestens eines Merkmalspunktes des Zielsubjekts unter Verwendung mindestens eines Zielerkennungsmodells beinhaltet:
Identifizieren, anhand des ersten optischen Bildes, des mindestens einen Merkmalspunktes des Zielsubjekts unter Verwendung des Merkmalspunkt-Erkennungsmodells.

6. System nach Anspruch 5, wobei das Identifizieren, anhand des ersten optischen Bildes, des mindestens einen Merkmalspunktes des Zielsubjekts unter Verwendung des Merkmalspunkt-Erkennungsmodells beinhaltet:
Bestimmen, basierend auf der mindestens einen Körperteilgrenze und des ersten optischen Bildes, eines zweiten optischen Bildes, das mindestens einen Körperteil des Zielsubjekts beinhaltet;
Identifizieren, anhand des zweiten optischen Bildes, des mindestens einen Merkmalspunktes des Zielsubjekts unter Verwendung des Merkmalspunkt-Erkennungsmodells.

7. System nach Anspruch 5, wobei das Merkmalspunkt-Erkennungsmodell beinhaltet:
ein Merkmalsextraktionsnetzwerk, das konfiguriert ist, um Merkmalskarten durch Extrahieren von Bildmerkmalen aus dem ersten optischen Bild, das in das Merkmalspunkt-Erkennungsmodell eingegeben wird, zu generieren; und
ein Probennetzwerk, das konfiguriert ist, um basierend auf den Merkmalskarten Informationen zu bestimmen, die sich auf den mindestens einen Merkmalspunkt des Zielsubjekts beziehen.

8. System nach einem der Ansprüche 5-7, wobei Identifizieren, anhand des ersten optischen Bildes, des mindestens einen Merkmalspunktes des Zielsubjekts unter Verwendung des Merkmalspunkt-Erkennungsmodells umfasst:
Erhalten, durch Eingeben des ersten optischen Bildes in das Merkmalspunkt-Erkennungsmodell, einer Heatmap, die eine Wahrscheinlichkeit angibt, dass jeder Punkt im ersten optischen Bild ein Merkmalspunkt des Zielsubjekts ist; und
Bestimmen, basierend auf der Heatmap, des mindestens einen Merkmalspunktes des Zielsubjekts.

9. System nach einem der Ansprüche 1 -8, wobei Identifizieren, anhand des ersten optischen Bildes basierend auf der mindestens einen Körperteilgrenze und des mindestens einen Merkmalspunktes, eines Bildbereichs, der dem Zielbereich entspricht, beinhaltet:
Bestimmen von Informationen, die sich auf den Zielbereich des Subjekts beziehen, basierend auf einem Untersuchungsziel des Zielsubjekts, wobei die Informationen, die sich auf den Zielbereich beziehen, mindestens eines von einem Körperteil des Zielsubjekts, der dem Zielbereich entspricht, oder einem Scanbereich des Zielbereichs beinhalten;
Bestimmen, basierend auf den Informationen, die sich auf den Zielbereich beziehen, mindestens einer Zielkörperteilgrenze aus der mindestens einen Körperteilgrenze und mindestens eines Zielmerkmalspunktes aus dem mindestens einen Merkmalspunkt; und
Bestimmen, basierend auf der mindestens einen Zielkörperteilgrenze und dem mindestens einen Zielmerkmalspunkt des Bildbereichs, wobei die mindestens eine erste Kante des Bildbereichs basierend auf der mindestens einen Zielkörperteilgrenze bestimmt wird und die mindestens eine zweite Kante des Bildbereichs basierend auf dem mindestens einen Zielmerkmalspunkt bestimmt wird.

10. System nach einem der Ansprüche 1-9, wobei das Bestimmen, basierend auf dem Bildbereich, ob ein Positionierungsvorgang der medizinischen Vorrichtung gestartet werden kann, beinhaltet:
Erhalten eines optischen Referenzbildes, das früher aufgenommen wurde als das erste optische Bild;
Bestimmen eines Referenzbildbereichs in dem optischen Referenzbild, der dem Zielbereich des Zielsubjekts entspricht; und
Bestimmen, basierend auf dem Referenzbildbereich und dem Bildbereich, ob der Positionierungsvorgang der medizinischen Vorrichtung gestartet werden kann.

11. System nach Anspruch 1, wobei das mindestens eine Zielerkennungsmodell Körperteilgrenzen-Erkennungsmodelle, die unterschiedlichen Körperteilgrenzen des Zielsubjekts entsprechen, und/oder unterschiedliche Merkmalspunkt-Erkennungsmodelle beinhaltet, die Merkmalspunkten entsprechen, die sich auf unterschiedlichen Körperteilen befinden.

12. System nach Anspruch 3, wobei das Backbone-Netzwerk mindestens ein Cross-Stage-Partial-, CSP-, Modul und ein Spatial-Pyramid-Convolutional-, SPC-, Modul beinhaltet, das mit dem mindestens einen CSP-Modul verbunden ist,
das mindestens eine CSP-Modul konfiguriert ist, um mindestens eine erste Merkmalskarte durch Extrahieren von Bildmerkmalen aus dem ersten optischen Bild zu erhalten; und
das SPC-Modul konfiguriert ist, um eine zweite Merkmalskarte durch Extrahieren von Bildmerkmalen mit unterschiedlichen Maßstäben aus dem ersten optischen Bild oder der mindestens einen ersten Merkmalskarte zu erhalten.

13. System nach Anspruch 1, wobei jede der mindestens einen Körperteilgrenze von einem Begrenzungsrahmen dargestellt wird, und
Informationen, die sich auf den Begrenzungsrahmen beziehen, Konfidenzkoeffizienteninformationen beinhalten, die dem Begrenzungsrahmen entsprechen, wobei die Konfidenzkoeffizienteninformationen, die dem Begrenzungsrahmen entsprechen, einen Konfidenzkoeffizienten beinhalten, der eine Zuverlässigkeit angibt, dass der Bereich des ersten optischen Bildes, der von dem Begrenzungsrahmen umschlossen ist, ein Vordergrundbereich oder ein Hintergrundbereich ist.

14. Verfahren, wobei das Verfahren auf einer Rechenvorrichtung implementiert wird, die mindestens eine Speichervorrichtung und mindestens einen Prozessor aufweist, wobei das Verfahren Folgendes umfasst:
Erhalten eines ersten optischen Bildes eines Zielsubjekts, das einen Zielbereich beinhaltet, der von einer medizinischen Vorrichtung zu scannen oder zu behandeln ist, wobei das erste optische Bild vor einem Scan oder einer Behandlung des Zielbereichs von einer Bilderfassungsvorrichtung aufgenommen wird;
Identifizieren, anhand des ersten optischen Bildes, mindestens einer Körperteilgrenze und mindestens eines Merkmalspunktes des Zielsubjekts unter Verwendung mindestens eines Zielerkennungsmodells, wobei jeder des mindestens einen Merkmalspunktes auf einen Punkt im ersten optischen Bild verweist, der einen Punkt von Interesse oder einen kritischen Punkt des Zielsubjekts darstellt; und
Identifizieren, anhand des ersten optischen Bildes, eines Bildbereichs, der dem Zielbereich des Zielsubjekts entspricht, basierend auf der mindestens einen Körperteilgrenze und dem mindestens einen Merkmalspunkt, wobei mindestens eine erste Kante des Bildbereichs basierend auf der mindestens einen Körperteilgrenze bestimmt wird, und mindestens eine zweite Kante des Bildbereichs basierend auf dem mindestens einen Merkmalspunkt bestimmt wird; und
Bestimmen, basierend auf dem Bildbereich, ob ein Positionierungsvorgang der medizinischen Vorrichtung gestartet werden kann.

15. System, umfassend:
ein Erfassungsmodul, das konfiguriert ist, um ein erstes optisches Bild eines Zielsubjekts zu erhalten, das einen Zielbereich beinhaltet, der von einer medizinischen Vorrichtung zu scannen oder zu behandeln ist, wobei das erste optische Bild vor einem Scan oder einer Behandlung des Zielbereichs von einer Bilderfassungsvorrichtung aufgenommen wird; und
ein Identifizierungsmodul, das konfiguriert ist, um:
anhand des ersten optischen Bildes mindestens eine Körperteilgrenze und mindestens einen Merkmalspunkt des Zielsubjekts unter Verwendung mindestens eines Zielerkennungsmodells zu identifizieren, wobei jeder des mindestens einen Merkmalspunktes auf einen Punkt im ersten optischen Bild verweist, der einen Punkt von Interesse oder einen kritischen Punkt des Zielsubjekts darstellt;
anhand des ersten optischen Bildes einen Bildbereich, der dem Zielbereich des Zielsubjekts entspricht, basierend auf der mindestens einen Körperteilgrenze und dem mindestens einen Merkmalspunkt zu identifizieren, wobei mindestens eine erste Kante des Bildbereichs basierend auf der mindestens einen Körperteilgrenze bestimmt wird, und mindestens eine zweite Kante des Bildbereichs basierend auf dem mindestens einen Merkmalspunkt bestimmt wird; und
basierend auf dem Bildbereich zu bestimmen, ob ein Positionierungsvorgang der medizinischen Vorrichtung gestartet werden kann.

## Revendications

1. Système, comprenant :
au moins un dispositif de stockage incluant un ensemble d'instructions pour imagerie médicale ; et
au moins un processeur en communication avec le au moins un dispositif de stockage, dans lequel, lors de l'exécution de l'ensemble d'instructions, le au moins un processeur est configuré pour ordonner au système d'effectuer des opérations incluant :
l'obtention d'une première image optique d'un sujet cible qui inclut une région cible à balayer ou à traiter par un dispositif médical, la première image optique étant capturée par un dispositif d'acquisition d'images avant un balayage ou un traitement de la région cible ;
l'identification, à partir de la première image optique, d'au moins une limite de partie du corps et d'au moins un point caractéristique du sujet cible à l'aide d'au moins un modèle de reconnaissance de cible, chacun du au moins un point caractéristique faisant référence à un point dans la première image optique qui représente un point d'intérêt ou un point critique du sujet cible ;
l'identification, à partir de la première image optique, d'une région d'image correspondant à la région cible du sujet cible en fonction de la au moins une limite de partie du corps et du au moins un point caractéristique, dans lequel au moins un premier bord de la région d'image est déterminé en fonction de la au moins une limite de partie du corps, et au moins un second bord de la région d'image est déterminé en fonction du au moins un point caractéristique ; et
la détermination, en fonction de la région d'image, pour établir si une procédure de positionnement du dispositif médical peut être démarrée.

2. Système selon la revendication 1, dans lequel le au moins un modèle de reconnaissance de cible inclut un modèle de reconnaissance de limite de partie du corps, et
l'identification, à partir de la première image optique, d'au moins une limite de partie du corps et d'au moins un point caractéristique du sujet cible à l'aide d'au moins un modèle de reconnaissance de cible inclut :
l'identification, à partir de la première image optique, de la au moins une limite de partie du corps du sujet cible à l'aide du modèle de reconnaissance de limite de parties du corps.

3. Système selon la revendication 2, dans lequel le modèle de reconnaissance de limite de partie du corps inclut :
un réseau fédérateur configuré pour générer des cartes de caractéristiques en extrayant des caractéristiques d'image de la première image optique ; et
un réseau de détection configuré pour déterminer des informations associées à la au moins une limite de partie du corps sur la base des cartes de caractéristiques.

4. Système selon la revendication 2 ou la revendication 3, dans lequel
le modèle de reconnaissance de limite de partie du corps est généré en formant un premier modèle préliminaire à l'aide d'une pluralité de premiers échantillons de formation, chacun de la pluralité de premiers échantillons de formation présente une première étiquette de formation incluant des informations de position d'échantillon, des informations de coefficient de confiance d'échantillon et des informations de classification d'échantillon relatives à une limite de partie du corps échantillon, et
une fonction de perte pour la formation du premier modèle préliminaire inclut au moins l'un des éléments suivants :
une première fonction de perte configurée pour évaluer un résultat prédit associé aux informations de position d'échantillon,
une deuxième fonction de perte configurée pour évaluer un résultat prédit associé aux informations du coefficient de confiance d'échantillon, ou
une troisième fonction de perte configurée pour évaluer un résultat prédit associé aux informations de classification d'échantillon.

5. Système selon la revendication 1, dans lequel le au moins un modèle de reconnaissance de cible inclut un modèle de reconnaissance de pointe caractéristiques, et
l'identification, à partir de la première image optique, d'au moins une limite de partie du corps et d'au moins un point caractéristique du sujet cible à l'aide d'au moins un modèle de reconnaissance de cible inclut :
l'identification, à partir de la première image optique, du au moins un point caractéristique du sujet cible à l'aide du modèle de reconnaissance de points caractéristiques.

6. Système selon la revendication 5, dans lequel l'identification, à partir de la première image optique, du au moins un point caractéristique du sujet cible à l'aide du modèle de reconnaissance de points caractéristiques inclut :
la détermination, sur la base de la au moins une limite de partie du corps et de la première image optique, d'une seconde image optique incluant au moins une partie du corps du sujet cible ;
l'identification, à partir de la seconde image optique, du au moins un point caractéristique du sujet cible à l'aide du modèle de reconnaissance de points caractéristiques.

7. Système selon la revendication 5, dans lequel le modèle de reconnaissance de points caractéristiques inclut :
un réseau d'extraction de caractéristiques configuré pour générer des cartes de caractéristiques en extrayant des caractéristiques d'image à partir de la première image optique entrée dans le modèle de reconnaissance de points caractéristiques ; et
un réseau d'échantillonnage configuré pour déterminer des informations relatives au au moins un point caractéristique du sujet cible sur la base des cartes de caractéristiques.

8. Système selon l'une quelconque des revendications 5-7, dans lequel l'identification, à partir de la première image optique, du au moins un point caractéristique du sujet cible à l'aide du modèle de reconnaissance de points caractéristiques comprend :
l'obtention, en entrant la première image optique dans le modèle de reconnaissance de points caractéristiques, d'une carte thermique indiquant une probabilité que chaque point dans la première image optique soit un point caractéristique du sujet cible ; et
la détermination, sur la base de la carte thermique, du au moins un point caractéristique du sujet cible.

9. Système selon l'une quelconque des revendications 1-8, dans lequel l'identification, à partir de la première image optique, d'une région d'image correspondant à la région cible sur la base de la au moins une limite de partie du corps et du au moins un point caractéristique inclut :
la détermination d'informations relatives à la région cible du sujet sur la base d'une cible d'examen du sujet cible, les informations relatives à la région cible incluant au moins un élément parmi une partie du corps du sujet cible correspondant à la région cible ou une plage de balayage de la région cible ;
la détermination, sur la base des informations relatives à la région cible, d'au moins une limite de partie de corps cible à partir de la au moins une limite de partie de corps et d'au moins un point caractéristique cible à partir du au moins un point caractéristique ; et
la détermination, sur la base de la au moins une limite de partie de corps cible et du au moins un point caractéristique cible, de la région d'image, dans lequel le au moins un premier bord de la région d'image est déterminé sur la base de la au moins une limite de partie de corps cible, et le au moins un second bord de la région d'image est déterminé sur la base du au moins un point caractéristique cible.

10. Système selon l'une quelconque des revendications 1-9, dans lequel la détermination, sur la base de la région d'image, pour établir si une procédure de positionnement du dispositif médical peut être démarrée inclut :
l'obtention d'une image optique de référence capturée plus tôt que la première image optique ;
la détermination d'une région d'image de référence dans l'image optique de référence correspondant à la région cible du sujet cible ; et
la détermination, sur la base de la région d'image de référence et de la région d'image, pour établir si la procédure de positionnement du dispositif médical peut être démarrée.

11. Système selon la revendication 1, dans lequel le au moins un modèle de reconnaissance de cible inclut des modèles de reconnaissance de limites de parties du corps correspondant à différentes limites de parties du corps du sujet cible et/ou différents modèles de reconnaissance de points caractéristiques correspondant à des points caractéristiques qui sont situés sur différentes parties du corps.

12. Système selon la revendication 3, dans lequel le réseau fédérateur inclut au moins un module partiel inter-étages, CSP, et un module convolutionnel à pyramide spatiale (SPC) connecté au au moins un module CSP,
le au moins un module CSP est configuré pour obtenir au moins une première carte de caractéristiques en extrayant des caractéristiques d'image de la première image optique ; et
le module SPC est configuré pour obtenir une seconde carte de caractéristiques en extrayant des caractéristiques d'image avec différentes échelles à partir de la première image optique ou de la au moins une première carte de caractéristiques.

13. Système selon la revendication 1, dans lequel chacune de la au moins une limite de partie du corps est représentée par un cadre de contour, et
des informations relatives au cadre de contour incluent des informations de coefficient de confiance correspondant au cadre de contour, les informations de coefficient de confiance correspondant au cadre de contour incluant un coefficient de confiance indiquant une fiabilité selon laquelle la région de la première image optique délimitée par le cadre de contour est une région de premier plan ou une région d'arrière-plan.

14. Procédé, le procédé étant mis en œuvre sur un dispositif informatique présentant au moins un dispositif de stockage et au moins un processeur, le procédé comprenant :
l'obtention d'une première image optique d'un sujet cible qui inclut une région cible à balayer ou à traiter par un dispositif médical, la première image optique étant capturée par un dispositif d'acquisition d'images avant un balayage ou un traitement de la région cible ;
l'identification, à partir de la première image optique, d'au moins une limite de partie du corps et d'au moins un point caractéristique du sujet cible à l'aide d'au moins un modèle de reconnaissance de cible, chacun du au moins un point caractéristique faisant référence à un point dans la première image optique qui représente un point d'intérêt ou un point critique du sujet cible ; et
l'identification, à partir de la première image optique, d'une région d'image correspondant à la région cible du sujet cible en fonction de la au moins une limite de partie du corps et du au moins un point caractéristique, dans lequel au moins un premier bord de la région d'image est déterminé en fonction de la au moins une limite de partie du corps, et au moins un second bord de la région d'image est déterminé en fonction du au moins un point caractéristiques ; et
la détermination, en fonction de la région d'image, pour établir si une procédure de positionnement du dispositif médical peut être démarrée.

15. Système, comprenant :
un module d'acquisition, configuré pour obtenir une première image optique d'un sujet cible qui inclut une région cible à balayer ou à traiter par un dispositif médical, la première image optique étant capturée par un dispositif d'acquisition d'images avant un balayage ou un traitement de la région cible ; et
un module d'identification, configuré pour :
identifier, à partir de la première image optique, au moins une limite de partie du corps et au moins un point caractéristique du sujet cible à l'aide d'au moins un modèle de reconnaissance de cible, chacun du au moins un point caractéristique faisant référence à un point dans la première image optique qui représente un point d'intérêt ou un point critique du sujet cible ;
identifier, à partir de la première image optique, une région d'image correspondant à la région cible du sujet cible en fonction de la au moins une limite de partie du corps et du au moins un point caractéristique, dans lequel au moins un premier bord de la région d'image est déterminé en fonction de la au moins une limite de partie du corps, et au moins un second bord de la région d'image est déterminé en fonction du au moins un point caractéristique ; et
déterminer, en fonction de la région de l'image, si une procédure de positionnement du dispositif médical peut être démarrée.
